# EUROPÄISCHE PATENTSCHRIFT

(11) **EP 3 817 706 B1**
(45) Veröffentlichungstag und Bekanntmachung des Hinweises auf die Patenterteilung: **11.03.2026**
(21) Anmeldenummer: 19739927.2
(22) Anmeldetag: 28.06.2019
(51) Int. Cl.: A61J 1/20

(54) **STOPFENVORRICHTUNG, MEDIKAMENTENBEHÄLTER UND VERFAHREN ZUR DURCHMISCHUNG ZWEIER SUBSTANZEN IN EINEM MEDIKAMENTENBEHÄLTER**
STOPPER DEVICE, MEDICAMENT CONTAINER, AND METHOD FOR MIXING TWO SUBSTANCES IN A MEDICAMENT CONTAINER
DISPOSITIF BOUCHON, RÉCIPIENT DE MÉDICAMENT ET PROCÉDÉ POUR LE MÉLANGE DE DEUX SUBSTANCES DANS UN RÉCIPIENT DE MÉDICAMENT

(30) Priorität: 03.07.2018 DE 102018210941
(43) Veröffentlichungstag der Anmeldung: 12.05.2021
(73) Patentinhaber: Vetter Pharma-Fertigung GmbH & Co. KG, 88212 Ravensburg (DE)
(72) Erfinder: MESSNER-RUGOVA, Fatbardha, 88074 Meckenbeuren (DE); EGGENSBERGER, Markus, 88427 Bad Schussenried (DE)
(74) Vertreter: Kordel, Mattias
(86) Internationale Anmeldenummer: PCT/EP2019/067479
(87) Internationale Veröffentlichungsnummer: WO 2020/007752

(56) Entgegenhaltungen:
- EP-A1- 2 399 564
- WO-A1-01/62175
- DE-A1- 3 635 574
- US-A- 2 688 966
- US-A- 4 116 240
- US-A- 4 254 768

## Beschreibung

Die Erfindung betrifft eine Stopfenvorrichtung, einen Medikamentenbehälter mit einer solchen Stopfenvorrichtung und ein Verfahren zur Durchmischung zweier strömungstechnisch voneinander getrennt gelagerten Substanzen in einem solchen Medikamentenbehälter.

Stopfenvorrichtungen, Medikamentenbehälter sowie Verfahren der hier angesprochenen Art sind bekannt. Dabei werden typischerweise in einem als Zweikammersystem ausgebildeten Medikamentenbehälter zwei strömungstechnisch getrennt voneinander gelagerte Substanzen miteinander vermischt. Eine erste Substanz der beiden Substanzen ist dabei in einer ersten, proximalen Kammer und eine zweite Substanz in einer zweiten, distalen Kammer angeordnet.

In einem solchen Medikamentenbehälter ist die erste, proximale Kammer in proximaler Richtung von einer ersten Stopfenvorrichtung, insbesondere Endstopfen, in distaler Richtung von einer zweiten Stopfenvorrichtung, insbesondere Mittelstopfen, und in radialer Richtung durch eine Innenwandung des Medikamentenbehälters begrenzt. Die zweite, distale Kammer ist in proximaler Richtung durch die zweite Stopfenvorrichtung, in radialer Richtung durch die Innenwandung des Medikamentenbehälters und in distaler Richtung von der Medikamentenbehälterspitze, welche vorzugsweise von einer Verschlusskappe verschlossen ist, begrenzt.

Vor einer Initialisierung des Medikamentenbehälters ist die proximale Kammer strömungstechnisch dicht gegenüber der distalen Kammer und einer Umgebung des Medikamentenbehälters verschlossen. Erst durch die Initialisierung wird die zweite Stopfeneinrichtung in eine Durchmischungsposition verlagert, wodurch eine strömungstechnische Verbindung zwischen den beiden Kammern über einen Bypass hergestellt wird, sodass sich die beiden Substanzen - solange die zweite Stopfenvorrichtung in der Durchmischungsposition angeordnet ist - miteinander vermischen können. Insbesondere wird durch das Ausüben von einem insbesondere manuellen Druck auf die erste Stopfenvorrichtung der Inhalt der proximalen Kammer, also die erste Substanz, aus der proximalen Kammer herausgedrückt und über den Bypass in die distale Kammer hineintransportiert, wobei sich das Volumen der proximalen Kammer verkleinert und die zweite Stopfenvorrichtung in der Durchmischungsposition verbleibt, bis die erste Stopfenvorrichtung gegen die zweite Stopfenvorrichtung stößt.

Die Initialisierung umfasst also eine Beaufschlagung der ersten Stopfeneinrichtung mit Druck in distale Richtung, wodurch eine Verlagerung der ersten Stopfenvorrichtung bewirkt wird. Aufgrund der Verlagerung und der strömungstechnischen Abdichtung der proximalen Kammer wird auch die zweite Stopfenvorrichtung mit Druck beaufschlagt, wodurch auch diese sich in distale Richtung bis in die Durchmischungsposition verlagert. In der Durchmischungsposition findet über den Bypass ein Druckausgleich statt, sodass die zweite Stopfenvorrichtung nicht mehr einseitig mit dem Druck beaufschlagt ist. Somit ist es bei solchen herkömmlichen Medikamentenbehältern zur Initialisierung und damit zur Durchmischung der beiden Substanzen erforderlich, dass beide Stopfenvorrichtungen verlagert werden.

Vor der Initialisierung, also der Anwendung des bekannten Verfahrens zur Durchmischung der Substanzen, sind die beiden Substanzen getrennt voneinander gelagert, wodurch die Sicherheit und/oder die Haltbarkeit der Substanzen erhöht sind. Erst vor einer geplanten Applikation, insbesondere Injektion, wird die Initialisierung durchgeführt, wobei die Stopfenvorrichtungen in dem Medikamentenbehälter in eine axiale Position verschoben wird - entlang einer Mittelachse des Medikamentenbehälters -, in welcher eine Durchmischung, insbesondere über einen Bypass möglich ist.

Um die Gleitfähigkeit der Stopfenvorrichtungen in dem Medikamentenbehälter zu erhöhen und damit einen sicheren Medikamentenbehälter, der eine aseptische Lagerung und/oder Applikation eines medizinischen Wirkstoffs sicherstellt, zu erreichen, ist die Innenwandung des Medikamentenbehälters silikonisiert. Dadurch ist es jedoch nicht möglich oder zumindest nicht sicher, in solchen bekannten Medikamentenbehältern Substanzen anzuordnen, die bei Kontakt mit der silikonisierten Innenwandung insbesondere ungewollt reagieren. Eine zeitlich und/oder räumlich voneinander getrennt stattfindende Befüllung der jeweiligen Kammern ist aufwendig, da der Medikamentenbehälter nach der Befüllung der ersten Kammer gedreht und/oder verschlossen werden und/oder die Befüllung der zweiten Kammer abgeschlossen sein muss, bevor die erste Kammer befüllt werden kann. Außerdem ist die Durchmischung der beiden Substanzen in einem solchen Medikamentenbehälter insbesondere aufgrund der typischerweise geringen Dimensionierung des Bypasses nicht optimal.

Zudem ist es mit solchen herkömmlichen Stopfenvorrichtungen nicht oder nur unter erschwerten Bedingungen möglich, eine Ampulle oder ein Injektionsfläschchen, auch Vial genannt, als Mehrkammersystem, insbesondere Doppelkammersystem, auszubilden, da diese Gefäße nur einendig offen ausgebildet sind. Somit kommt es beim Verlagern von axial abdichtenden Stopfenvorrichtungen in solchen Gefäßen zu Druckschwankungen, welche eine Verlagerung in axialer Richtung erschweren und letztlich limitieren. Insbesondere die als Mittelstopfen fungierende zweite Stopfenvorrichtung kann nicht ohne weiteres weit genug in ein solches Gefäß hineinverlagert werden. Zudem ist in solchen Gefäßen das Verlagern der zweiten Stopfenvorrichtung in die Durchmischungsposition aufgrund der dadurch entstehenden Druckschwankungen nicht möglich oder zumindest deutlich erschwert und somit nicht praktikabel.

Eine Stopfenvorrichtung gemäß dem Oberbegriff des Anspruchs 1 ist in US 2 688 966 A offenbart. Weitere Stopfenvorrichtungen sind offenbart in DE 36 35 574 A1 und US 4 254 768 A.

Aufgabe der Erfindung ist es, eine Stopfenvorrichtung, einen Medikamentenbehälter und ein Verfahren zur Durchmischung zweier Substanzen in einen Medikamentenbehälter zu schaffen, wobei die genannten Nachteile vermieden werden. Insbesondere ist es Aufgabe der Erfindung einen Medikamentenbehälter und eine Stopfenvorrichtung zu schaffen, in welchem instabile Substanzen, insbesondere Pulverarzneien, die nicht mit einer silikonisierten Innenwandung in Berührung kommen dürfen, sicher gelagert und zur Durchmischung mit einer zweiten Substanz gebracht werden können.

Die Aufgabe wird gelöst, indem eine Stopfenvorrichtung gemäß Anspruch 1 zur Abdichtung eines Medikamentenbehälters geschaffen wird. Weitere Ausgestaltungen sind in den abhängigen Ansprüchen beschrieben.

Die Stopfenvorrichtung weist ein Stopfenelement auf, wobei die Stopfenvorrichtung ausgebildet ist, den Medikamentenbehälter in einer axialen Richtung einer Mittelachse des Medikamentenbehälters insbesondere mittels des Stopfenelements abzudichten, wobei die Stopfenvorrichtung in den Medikamentenbehälter zumindest teilweise einführbar und das Stopfenelement bei bestimmungsgemäßer Einbaulage in dem Medikamentenbehälter entlang der Mittelachse des Medikamentenbehälters verlagerbar ausgebildet ist, und wobei die Stopfenvorrichtung, insbesondere das Stopfenelement, einen Haltebereich mit zumindest einem Haltemittel aufweist. Die Stopfenvorrichtung zeichnet sich dadurch aus, dass ein Verschlusselement - in einem ersten Funktionszustand der Stopfenvorrichtung - mittels des Haltemittels ortsfest zu dem Stopfenelement, insbesondere unmittelbar an dem Stopfenelement, in einer Halteposition lösbar derart gehalten ist, dass zwischen dem Verschlusselement einerseits und dem Stopfenelement andererseits ein Hohlraum ausgebildet ist. Somit kann in diesem Hohlraum der Stopfenvorrichtung eine insbesondere zur Durchmischung vorgesehene Substanz angeordnet und gegenüber der Umgebung abgedichtet gelagert werden, ohne dass hierzu ein Medikamentenbehälter notwendig ist. Die Befüllung eines Medikamentenbehälters mit zwei Substanzen kann auf einfache Weise in zwei separaten Schritten, die voneinander größtenteils unabhängig sind, erfolgen. Auch ist es möglich in einer solchen Stopfenvorrichtung eine instabile Substanz, insbesondere Pulverarznei, sicher zu lagern, da ein Kontakt mit Silikon, insbesondere einer silikonisierten Innenwandung eines Medikamentenbehälters, vermieden ist. Mittels einer solchen Stopfenvorrichtung ist es zudem möglich und vereinfacht, ein Doppelkammersystem in einem Injektionsfläschchen und/oder einer Ampulle auszubilden, da kein Mittelstopfen zur Trennung von zwei Kammern erforderlich ist.

Unter einer Substanz wird hier insbesondere eine medizinische Substanz, ganz besonders ein medizinischer Wirkstoff und/oder Hilfsstoff verstanden.

Die Stopfenvorrichtung, insbesondere das Verschlusselement und das Stopfenelement, ist/sind eingerichtet, um in dem Hohlraum eine solche Substanz, insbesondere einen medizinischen Wirkstoff, vorzugsweise aspetisch aufzunehmen, wobei der Hohlraum vorzugsweise strömungstechnisch dicht ausgebildet ist. Vorzugsweise weist die Stopfenvorrichtung einen Kunststoff auf oder besteht aus Kunststoff.

Unter einem Stopfenelement wird hier ein Stopfen, insbesondere Endstopfen, für einen Medikamentenbehälter verstanden. Das Stopfenelement weist vorzugsweise ein elastisches Material auf oder besteht aus einem solchen Material. Besonders bevorzugt besteht das Stopfenelement aus einem Elastomer und/oder Weichplastik. In einem Dichtungsbereich ist das Stopfenelement vorzugsweise zumindest teilweise elastisch ausgebildet und/oder entlang einer Umfangsfläche in dem Dichtungsbereich, welche bei bestimmungsgemäßer Einbaulage in einem Medikamentenbehälter einer Innenwandung des Medikamentenbehälters und/oder einer Einsteckhülse zugewandt ist und an dieser dicht anliegt, zur abdichtenden Anlage an der Innenwandung und/oder der Einsteckhülse eingerichtet. Der Medikamentenbehälter wird also in axialer Richtung insbesondere durch die dichte Anlage des Stopfenelements und/oder der Stopfenvorrichtung an einer Innenwandung des Medikamentenbehälters abgedichtet, sodass zumindest eine zur Aufnahme einer Substanz eingerichtete Kammer des Medikamentenbehälters in axiale, vorzugsweise proximale Richtung begrenzt ist.

Unter einem Medikamentenbehälter wird hier insbesondere eine Spritze, Karpule, Ampulle und/oder Injektionsfläschchen, sogenanntes Vial, verstanden. Derartige Medikamentenbehälter, insbesondere Spritzen und Karpulen, sind dabei als Einkammersystem oder als Mehrkammersystem, insbesondere Doppelkammersystem, ausgebildet.

Die Stopfenvorrichtung und/oder das Stopfenelement sind vorzugsweise zumindest teilweise, besonders bevorzugt vollständig in den Medikamentenbehälter einführbar. In dem eingeführten Zustand, also bei bestimmungsgemäßer Einbaulage in den Medikamentenbehälter, ist das Stopfenelement vorzugsweise mittels einer Kolbenstange und/oder einer Ausbringvorrichtung entlang der Mittelachse verlagerbar.

Das Haltemittel in dem Haltebereich ist vorzugsweise einstückig mit dem Stopfenelement ausgebildet. Vorzugsweise weist das Haltemittel einen in radialer Richtung elastischen Bereich auf, sodass das Verschlusselement - in dem ersten Funktionszustand - mittels radialer Haltekräfte gehalten ist. Besonders bevorzugt bestehen das Haltemittel und das Stopfenelement aus einem elastischen Material, insbesondere Gummi und/oder Weichplastik.

Das Verschlusselement wird in dem ersten Funktionszustand vorzugsweise derart in der Halteposition gehalten, dass eine Verlagerung des Stopfenelements nicht ohne eine gleichzeitige Verlagerung des Verschlusselements möglich ist. Vorzugsweise besteht also zwischen dem Stopfenelement und dem Verschlusselement zumindest im ersten Funktionszustand der Stopfenvorrichtung ein mechanischer Kontakt.

Gemäß einer Weiterbildung der Erfindung ist vorgesehen, dass der Hohlraum im ersten Funktionszustand strömungstechnisch gegen eine Wandung, insbesondere Innenwandung, des Medikamentenbehälters abgedichtet ist. Somit ist ein Kontakt mit einer silikonisierten Fläche vermieden und die Sicherheit für eine in dem Hohlraum gelagerte Substanz ist hoch.

Der Hohlraum ist - in dem ersten Funktionszustand - vorzugsweise ausschließlich von dem Verschlusselement und dem Stopfenelement begrenzt und dadurch ausgebildet. Dabei ist insbesondere vorgesehen, dass der Hohlraum von einer Aushöhlung in dem Stopfenelement und/oder in dem Verschlusselement gebildet ist, wobei der Hohlraum mittels des Verschlusselements verschließbar, insbesondere in dem ersten Funktionszustand verschlossen ist. Das Verschlusselement dichtet also den Hohlraum ab, sodass eine darin gelagerte Substanz strömungstechnisch gegenüber einer Umgebung, insbesondere dem Medikamentenbehälter, abgedichtet ist. Insbesondere ist somit eine strömungstechnische Abdichtung gegenüber einer Innenwandung eines Medikamentenbehälters - bei bestimmungsgemäßer Einbaulage in dem Medikamentenbehälter - geschaffen. Somit ist auch bereits ohne Medikamentenbehälter ein dichter Hohlraum zur sicheren Lagerung einer Substanz geschaffen.

Gemäß einer Weiterbildung der Erfindung ist vorgesehen, dass der Hohlraum in einem zweiten Funktionszustand offen ist, wobei das Verschlusselement nicht von dem Haltemittel in der Halteposition gehalten ist. Somit ist es möglich, den Hohlraum zu öffnen und damit eine darin gelagerte erste Substanz zur Durchmischung mit einer in einer Kammer eines Medikamentenbehälters gelagerten, zweiten Substanz zu bringen. Bei bestimmungsgemäßer Einbaulage der Stopfenvorrichtung in dem Medikamentenbehälter ist der Hohlraum in dem zweiten Funktionszustand also insbesondere gegenüber der Kammer des Medikamentenbehälters offen, wobei eine strömungstechnische Verbindung zwischen dem Hohlraum und der Kammer, in welcher vorzugsweise die zweite Substanz gelagert ist, besteht. Somit ist eine Durchmischung von zwei Substanzen, insbesondere der ersten Substanz und der zweiten Substanz, in einem Medikamentenbehälter mit einer solchen Stopfenvorrichtung einfach und sicher durchführbar.

Alternativ ist es jedoch auch möglich, dass das Verschlusselement in dem zweiten Funktionszustand nicht gänzlich von dem Haltemittel losgelassen wird, sondern stattdessen weiterhin zumindest teilweise an dem Stopfenelement, insbesondere in dem Haltebereich, gehalten ist, wobei das Verschlusselement den Hohlraum jedoch nicht mehr abdichtet, sodass eine strömungstechnische Verbindung nach außerhalb des Hohlraums, insbesondere in die Kammer des Medikamentenbehälters, besteht.

Alternativ oder zusätzlich ist vorgesehen, dass auch in einem nicht in den Medikamentenbehälter eingeführten Zustand der Stopfenvorrichtung das Verschlusselement in dem ersten Funktionszustand ortsfest zu dem Stopfenelement gehalten ist. Somit ist die Stopfenvorrichtung flexibel einsetzbar und ein Medikamentenbehälter zur sicheren Lagerung der ersten Substanz in der Stopfenvorrichtung nicht erforderlich.

Besonders bevorzugt ist das Stopfenelement der Stopfenvorrichtung als Endstopfen für einen Medikamentenbehälter ausgebildet, wobei vorgesehen ist, dass die Stopfenvorrichtung und - zumindest in dem ersten Funktionszustand - das Stopfenelement endständig, insbesondere am proximalen Ende einer Kammer eines Medikamentenbehälters, angeordnet ist. Unter einem endständig angeordneten Endstopfen in einem Medikamentenbehälter wird hier insbesondere ein den Medikamentenbehälter abdichtender Stopfen verstanden, wobei ausgehend von dem Stopfen in distale Richtung sämtliche von dem Medikamentenbehälter umfasste insbesondere medizinische Substanzen und/oder sämtliche dafür eingerichtete Kammern angeordnet sind. Ausgehend von dem endständig angeordneten Endstopfen in proximaler Richtung sind dagegen keine Kammern oder Bereiche vorgesehen, welche eingerichtet sind, um eine Substanz, insbesondere medizinische Substanz, sicher zu lagern. Somit trennt ein solcher Endstopfen einen distal des Endstopfens vorgesehenen aseptischen Bereich von einem proximal des Endstopfens vorgesehen Bereich, welcher nur geringeren, insbesondere keinen Reinheitsanforderungen unterliegt.

Gemäß der Erfindung ist vorgesehen, dass das Verschlusselement als Mischkörper, als Kugel, für den Medikamentenbehälter ausgebildet ist.

Gemäß einer Weiterbildung der Erfindung ist vorgesehen, dass das Verschlusselement im zweiten Funktionszustand - insbesondere unabhängig von dem Stopfenelement frei beweglich in dem Medikamentenbehälter angeordnet ist. Vorzugsweise ist das kugelförmige Verschlusselement in dem zweiten Funktionszustand lose in dem Medikamentenbehälter angeordnet. Damit ist die Durchmischung von der ersten Substanz mit der zweiten Substanz durch Schütteln des Medikamentenbehälters, wobei der Mischkörper in dem Medikamentenbehälter zusätzliche Turbulenzen verursacht, verbessert.

Gemäß der Erfindung ist vorgesehen, dass das Stopfenelement - in dem ersten Funktionszustand - auf seiner dem Verschlusselement zugewandten Seite einen zumindest teilweise in Richtung des Verschlusselements ausgerichteten Ausbringvorsprung aufweist. Der Ausbringvorsprung kann zwar zumindest in eine Längsrichtung entlang einer Mittelachse bei bestimmungsgemäßer Einbaulage in einem Medikamentenbehälter elastisch ausgebildet sein, ist jedoch derart stabil ausgebildet, dass das Verschlusselement von dem Ausbringvorsprung aus der Halteposition, in welcher der Hohlraum dicht verschlossen ist, heraus in eine axiale Richtung, insbesondere in distaler Richtung des Medikamentenbehälters verlagert werden kann, wobei die von dem Haltemittel aufgebrachten Haltekräfte, überwunden werden. Eine axiale Widerstandskraft, also die Festigkeit des Ausbringvorsprungs, welche einer axialen Verformung des Ausbringvorsprungs entgegenwirkt, ist also größer als die von dem Haltemittel aufgebrachte Haltekraft.

Eine zum Ausbringen des Verschlusselements aus der Halteposition notwendige axiale Kraft, insbesondere in distaler Richtung, wird vorzugsweise von einer Kolbenstange und einem manuellen Druck auf die Kolbenstange aufgebracht. Der Ausbringvorsprung springt vorzugsweise in distaler Richtung vor. Somit kann das Verschlusselement auch nach einer Positionierung in dem Medikamentenbehälter auf einfache Weise aus der Halteposition heraus verlagert werden und die Stopfenvorrichtung somit in den zweiten Funktionszustand überführt werden. Dabei ist stets der Hohlraum und die darin angeordnete Substanz und - bei bestimmungsgemäßer Einbaulage in einem Medikamentenbehälter - die Kammer des Medikamentenbehälters und die darin gelagerte zweite Substanz insbesondere steril gegenüber einer Umgebung des insbesondere mit einer Verschlusskappe verschlossenen Medikamentenbehälters strömungstechnisch abgedichtet.

Vorzugsweise ist - bei bestimmungsgemäßer Anordnung in dem rotationssymmetrischen, insbesondere zumindest im Wesentlichen zylindrischen Medikamentenbehälter - der Ausbringvorsprung konzentrisch zu einer Längsachse des Medikamentenbehälters angeordnet. Somit sind die aufzubringenden Kräfte in distaler Richtung minimal und die Abdichtung zwischen der Stopfenvorrichtung und einer Innenwandung des Medikamentenbehälters aufgrund der dadurch ausgebildeten Symmetrie besonders hoch.

Bevorzugt ist eine Außenwandung des Stopfenelements im Haltebereich radial nach innen versetzt im Vergleich zu einer Außenwandung in dem Dichtungsbereich, sodass hier - bei bestimmungsgemäßer Einbaulage in einem Medikamentenbehälter - ein in Umfangsrichtung umlaufender Freiraum zwischen Stopfenelement und Innenwandung des Medikamentenbehälters zum Ausweichen für das Haltemittel ausgebildet ist. Aufgrund des Freiraums ist die Ausbringung des Verschlusselements aus dem Haltebereich vereinfacht, da das Material des Stopfenelements im Haltebereich weniger stark komprimiert, sondern stattdessen nur in den Freiraum hinein verformt werden muss.

Gemäß einer Weiterbildung der Erfindung ist vorgesehen, dass der Ausbringvorsprung - zumindest in einem Auswurfszustand der Stopfenvorrichtung, vorzugsweise aber auch bereits in dem ersten Funktionszustand - mit einer dem Verschlusselement zugewandten Seite und/oder Spitze und/oder Stirnfläche an dem Verschlusselement anliegt. Der Auswurfszustand bezeichnet somit den Zustand der Stopfenvorrichtung, in welchem sich das Verschlusselement gerade noch in dem ersten Funktionszustand befindet, jedoch durch eine minimale Kraft auf die Stopfenvorrichtung in distaler Richtung und einem dadurch ausgeübten Druck von dem Ausbringvorsprung auf das Verschlusselement unmittelbar in den zweiten Funktionszustand übergehen würde. Der Auswurfszustand ist somit der Grenzzustand zwischen dem ersten Funktionszustand und dem zweiten Funktionszustand. Somit sind die aufzubringenden Kräfte in distaler Richtung minimal und die Ausbringung des Verschlusselements aus dem Haltebereich erfolgt besonders kontrolliert.

Gemäß einer Weiterbildung der Erfindung ist vorgesehen, dass das Verschlusselement zumindest ein erstes Rastmittel aufweist, wobei das Haltemittel der Stopfenvorrichtung zumindest ein zu dem ersten Rastmittel korrespondierend ausgebildetes zweites Rastmittel aufweist, welches in dem Haltebereich an einer dem Verschlusselement zugewandten Seite des Stopfenelements angeordnet ist, wobei das erste Rastmittel und das zweite Rastmittel - in dem ersten Funktionszustand - rastend ineinandergreifen. Somit ist das Verschlusselement sicher an dem Stopfenelement gehalten.

Vorzugsweise weist das Verschlusselement zumindest eine Rastaufnahme, insbesondere eine ringförmig umlaufende Rastnut, auf, wobei das Haltemittel der Stopfenvorrichtung zumindest einen zu der Rastaufnahme korrespondierend ausgebildeten Rastvorsprung aufweist, welcher in dem Haltebereich an einer dem Verschlusselement zugewandten Seite des Stopfenelements angeordnet ist, wobei der Rastvorsprung - in dem ersten Funktionszustand - in die Rastaufnahme eingreift. In diesem Fall ist also das erste Rastmittel als Rastaufnahme und das zweite Rastmittel als Rastvorsprung ausgebildet. Vorzugsweise ist die Rastaufnahme - bei bestimmungsgemäßer Einbaulage der Stopfenvorrichtung in dem Medikamentenbehälter - in Umfangsrichtung um eine Mittelachse des Medikamentenbehälters herum ausgebildet. Somit ist das Verschlusselement besonders zuverlässig in der Halteposition gehalten.

Alternativ ist es aber auch möglich, dass das erste Rastmittel als Rastvorsprung und das zweite Rastmittel als Rastaufnahme ausgebildet ist, sodass das Haltemittel die Rastaufnahme und das Verschlusselement den Rastvorsprung aufweist. Auch in diesem Fall ist das Verschlusselement sicher in der Halteposition gehalten.

Gemäß einer Weiterbildung der Erfindung ist vorgesehen, dass die Stopfenvorrichtung eine das Stopfenelement, insbesondere in Umfangsrichtung, umhüllende Einsteckhülse aufweist, wobei bevorzugt eine Außenseite einer Hülsenwand zur dichten Anlage an einer Innenwandung des Medikamentenbehälters ausgebildet ist. Das Stopfenelement liegt hierbei dicht an einer der Außenseite gegenüberliegenden Innenseite der Hülsenwand an. Vorzugsweise ist die Einsteckhülse rotationssymmetrisch, insbesondere zumindest abschnittsweise zylinderförmig ausgebildet. Die Hülsenwand weist in radialer Richtung eine endliche, bestimmte Dicke auf, sodass am distalen Ende der Einsteckhülse bei bestimmungsgemäßer Einbaulage der Stopfenvorrichtung in dem Medikamentenbehälter in distaler Richtung ein zurückspringender, in radialer Richtung aufweitender Bereich gebildet ist. Vorzugsweise ist der Haltebereich der Stopfenvorrichtung in dem eingeführten Zustand in die Einsteckhülse radial nach innen vorgespannt, indem der Haltebereich von der Einsteckhülse radial zusammengedrückt ist. Nach einer Verlagerung des Haltebereichs in diesen zurückspringenden Bereich ist der Haltebereich weniger stark vorgespannt, vorzugsweise entspannt. Somit weitet sich der Haltebereich in radialer Richtung nach außen auf, und das Verschlusselement ist nicht länger gehalten. Insbesondere löst sich der Rastvorsprung in dieser Verlagerungsstellung des Stopfenelements beziehungsweise des Haltebereichs aus der Rastaufnahme heraus, sodass die hierdurch gebildete formschlüssige Befestigung des Verschlusselements an dem Stopfenelement aufgehoben ist. Somit ist ein besonders einfach betätigbarer Freigabemechanismus für das Verschlusselement geschaffen, mit dessen Hilfe insbesondere auch in einem zylindersymmetrisch ausgebildeten Medikamentenbehälter ohne Bypass zwei Substanzen zur Durchmischung gebracht werden können, wobei lediglich das Stopfenelement in distale Richtung verlagert werden muss.

Die Einsteckhülse der Stopfenvorrichtung ist an ihrem distalen Ende, insbesondere im zurückspringenden Bereich, vorzugsweise keilförmig ausgebildet, wobei sie insbesondere eine - in Umfangsrichtung umlaufende - Ablaufschräge für das Stopfenelement aufweist, sodass die radiale Aufweitung im zurückspringenden Bereich gleichmäßig und ohne Sprünge erfolgt. Somit ist die Herstellung der Stopfenvorrichtung, wobei das Verschlusselement in eine dem ersten Funktionszustand zugeordnete, erste Funktionsstellung verlagert wird, vereinfacht. Auch sind damit die Sicherheit, insbesondere die Abdichtung des Hohlraums und/oder der Kammer des Medikamentenbehälters, sowie die Handhabung bei der Durchmischung und/oder Ausbringung der durchmischten Substanzen verbessert.

Gemäß einer Weiterbildung der Erfindung ist vorgesehen, dass die Einsteckhülse als Fingerauflage ausgebildet ist und an einem proximalen Ende zumindest eine radial abstehende Fingerplatte aufweist, welche von einem Finger hintergreifbar ausgebildet ist. Die Fingerplatte ist vorzugsweise vollständig um eine Mittelachse umlaufend oder in zumindest zwei einzelne, gegenüberliegende Platten aufgeteilt ausgebildet. Wichtig ist, dass die Fingerplatte insbesondere gegenüber einer Außenwandung eines Medikamentenbehälters, in welcher die Stopfenvorrichtung anordenbar ist, radial absteht, sodass sie hintergreifbar ist und damit die Handhabung des Medikamentenbehälters vereinfacht ist.

Gemäß einer Weiterbildung der Erfindung ist vorgesehen, dass die Einsteckhülse eine Befestigungsstruktur zur Befestigung der Einsteckhülse an einem Medikamentenbehälter aufweist, wobei die Befestigungsstruktur vorzugsweise eine Aufnahme für den Medikamentenbehälter aufweist, in welcher der Medikamentenbehälter mit seinem proximalen Ende, insbesondere eine proximale, radiale Verdickung des Medikamentenbehälters, rastend und/oder klemmend aufnehmbar ist. Vorzugsweise ist die Befestigungsstruktur von einer sich axial erstreckenden, um die Mittelachse umlaufenden, in distaler Richtung offen ausgebildete Aussparung gebildet, in welche das proximale Ende des Medikamentenbehälters einführbar ist, wobei es schließlich zur rastenden und/oder klemmenden Aufnahme gebracht ist. Somit ist die Stopfenvorrichtung einfach und schnell an einem Medikamentenbehälter anbringbar.

Das Stopfenelement, insbesondere das Haltemittel, ist in dem Haltebereich vorzugsweise - bei bestimmungsgemäßer Einbaulage in einem Medikamentenbehälter - beabstandet zu einer Innenwandung des Medikamentenbehälters und/oder zu einer Innenseite der Einsteckhülse angeordnet. Somit ist es möglich, dass das Haltemittel in dem Haltebereich insbesondere bei einer Krafteinwirkung auf das Stopfenelement radial auseinandergedrängt wird und das Verschlusselement dadurch freigegeben wird.

Die Aufgabe wird auch gelöst, indem ein Medikamentenbehälter gemäß Anspruch 10 mit einem vorzugsweise zylindrischen Grundkörper, welcher insbesondere aus Glas besteht, und mit einer erfindungsgemäßen Stopfenvorrichtung oder einer Stopfenvorrichtung gemäß einer oder mehreren der zuvor beschriebenen Ausführungsformen geschaffen wird. Weitere Ausgestaltungen sind in den abhängigen Ansprüchen beschrieben.

Die Stopfenvorrichtung ist derart in den Medikamentenbehälter hinein verlagert angeordnet, dass das Stopfenelement ein proximales Ende des Medikamentenbehälters gegenüber einer insbesondere dicht verschließbaren Kammer des Grundkörpers abdichtet, wobei die Kammer in radialer Richtung von einer Innenwandung des Grundkörpers und in distaler Richtung von einer verschließbaren Auslassöffnung des Grundkörpers und/oder einer Verschlusskappe begrenzt ist. Auch die Kammer ist vorzugsweise rotationssymmetrisch, im Wesentlichen zylindrisch ausgebildet. Die Kammer ist von der Stopfenvorrichtung aus betrachtet in einer distalen Richtung des Medikamentenbehälters vorgesehen und zur Aufnahme einer medizinischen Substanz, insbesondere eines medizinischen Wirk- und/oder Hilfsstoffs, ganz besonders eines Lösungsmittels für eine Pulverarznei, eingerichtet.

Besonders bevorzugt ist somit durch die Kammer ein abgeschlossenes, insbesondere steriles Volumen zur Aufnahme der zweiten Substanz ausgebildet. Im zweiten Funktionszustand der Stopfenvorrichtung ist das Verschlusselement der Stopfenvorrichtung insbesondere lose in dieser Kammer angeordnet. Vorzugsweise ist das Verschlusselement größer als die Auslassöffnung des Grundkörpers, sodass das Verschlusselement nicht durch die Auslassöffnung aus der Kammer herausverlagert werden kann. Das Verschlusselement ist somit als Mischkörper ausgebildet.

Gemäß einer Weiterbildung der Erfindung ist vorgesehen, dass die Auslassöffnung des Grundkörpers zumindest auf ihrer der Kammer zugewandten Seite eine Geometrie, insbesondere einen kreuzförmigen Querschnitt, aufweist, welche von einer der Auslassöffnung zugewandten Seite, insbesondere von jeder Seite des Verschlusselements derart abweicht, dass ein strömungstechnisch dichter Verschluss der Auslassöffnung durch das Verschlusselement verhindert ist. Vorzugsweise ist das Verschlusselement dabei rund, insbesondere kugelförmig, ausgebildet. Somit ist verhindert, dass die Auslassöffnung durch das Verschlusselement - in dem zweiten Funktionszustand - verstopft werden kann. Insbesondere ist das Verschlusselement ungeeignet, die Auslassöffnung strömungstechnisch dicht zu verschließen, sodass ein Ausbringen der ersten und/oder zweiten Substanz aus dem Medikamentenbehälter - insbesondere in dem zweiten Funktionszustand - nicht von dem Verschlusselement verhindert werden kann.

Vorzugsweise ist eine radiale Ausdehnung der unrunden Geometrie größer als eine radiale Erstreckung einer zur Anlage an der Auslassöffnung geeigneten Seite des Verschlusselements. Alternativ oder zusätzlich weist das Verschlusselement an seiner Außenfläche eine Krümmung auf, die von einer Krümmung einer die Auslassöffnung aufweisenden Bodenwandung des Medikamentenbehälters verschieden ist.

Sofern das Verschlusselement Rastaufnahmen aufweist, ist das Verschlusselement mit Ausnahme dieser Rastaufnahmen rund, insbesondere kugelförmig ausgebildet. Insbesondere ist dabei vorgesehen, dass die ringförmig ausgebildete Rastnut äquatorial auf dem kugelförmig ausgebildeten Verschlusselement angeordnet ist, wobei ein oberer Halbkugelabschnitt des Verschlusselements von einem unteren Halbkugelabschnitts des Verschlusselements durch die Rastnut getrennt wird.

Vorzugsweise ist die Verschlusskappe distal am Grundkörper zum Verschließen der Auslassöffnung angeordnet. Unter einer Verschlusskappe wird hier ein die Kammer verschließendes Element, insbesondere auch eine verschlossene Injektionsvorrichtung verstanden.

In dem Hohlraum der Stopfenvorrichtung ist hier eine erste Substanz angeordnet, wobei in der Kammer des Medikamentenbehälters eine zweite Substanz angeordnet ist, wobei diese beiden Substanzen zur Durchmischung bestimmt sind und der Zeitpunkt der Durchmischung von einem Anwender wählbar ist, da die beiden Substanzen in voneinander strömungstechnisch abgedichteten Volumina, nämlich der Kammer des Medikamentenbehälters und dem Hohlraum der Stopfenvorrichtung, angeordnet sind, wobei die Abdichtung zwischen der Kammer und dem Hohlraum von dem Anwender durch Druck auf die Stopfenvorrichtung aufgehoben werden kann.

Vorzugsweise weist der Medikamentenbehälter eine Fingerauflage auf, welche geeignet ist, um bei Ausbringung der Wirksubstanzen aus dem Medikamentenbehälter eine geeignete Kraft auf eine Ausbringvorrichtung, insbesondere eine Kolbenstange, aufzubringen. Die Fingerauflage ist dabei einstückig mit dem Grundkörper oder - sofern die Stopfenvorrichtung in einer die Fingerplatte aufweisenden Einsteckhülse angeordnet ist - separat zu dem Grundkörper ausgebildet. In beiden Fällen ist die Fingerauflage jedoch fest am Grundkörper gehalten, sodass die Anwendung, insbesondere die Ausbringung einer Substanz, insbesondere beider Substanzen, aus dem Medikamentenbehälter bei beiden Ausführungsbeispielen gleichartig erfolgt.

Gemäß einer Weiterbildung der Erfindung ist vorgesehen, dass eine Ausbringvorrichtung, insbesondere Kolbenstange vorgesehen ist, wobei am distalen Ende der Ausbringvorrichtung eine konvexe, insbesondere kugel-, konus- und/oder kegelförmige Hervorspringung vorgesehen ist. Unter dem distalen Ende wird hier das der Stopfenvorrichtung zugewandte Ende der Ausbringvorrichtung verstanden, welches beim Ausbringen der Substanzen aus dem Medikamentenbehälter unmittelbar auf das insbesondere proximale Ende der Stopfenvorrichtung einwirkt. Vorzugsweise ist die Hervorspringung relativ zu der Mittelachse des Medikamentenbehälters zentriert, sodass die Ausbringvorrichtung beim Einwirken auf die Stopfenvorrichtung diese in deren zentralen Bereich mit einer maximalen Kraft beaufschlagt.

Gemäß einer Weiterbildung der Erfindung ist vorgesehen, dass das Stopfenelement elastisch ausgebildet ist, wobei es mittels der Hervorspringung der Ausbringvorrichtung durch insbesondere manuelle Druckausübung in distaler Richtung auf die Ausbringvorrichtung verformbar ist, wobei der Ausbringsvorsprung des Stopfenelements eine Festigkeit in axialer Richtung aufweist, die größer ist als es dem zur Ausbringung des Verschlusselements erforderlichen Druck entspricht. Die Festigkeit in axialer Richtung des Stopfenelements ist aufgrund von dessen struktureller Ausbildung und/oder des Materials realisiert. Insbesondere ist der Ausbringvorsprung hierbei konusförmig ausgebildet. Somit ist eine einfache Ausbringung des Verschlusselements aus dem Haltebereich möglich.

Unter einer Festigkeit wird hier insbesondere ein mechanischer Widerstand verstanden, welcher ein elastisches und/oder plastisches Materialversagen verhindert. Vorzugsweise lässt sich die Festigkeit als mechanische Spannung, insbesondere als Druck, oder als Kraft quantifizieren und vorzugsweise in einen Druck umrechnen. In diesem Fall ist die Festigkeit in axialer Richtung vorzugsweise größer als der zur Ausbringung des Verschlusselements erforderliche Druck. Die Festigkeit des Ausbringvorsprungs verhindert somit insbesondere eine elastische und/oder plastische Verformung des Ausbringvorsprungs.

Die Aufgabe wird auch gelöst, indem ein Verfahren gemäß Anspruch 14 zur Durchmischung zweier strömungstechnisch voneinander getrennt gelagerter Substanzen in einem erfindungsgemäßen Medikamentenbehälter oder einem Medikamentenbehälter gemäß einer oder mehreren der zuvor beschriebenen Ausführungsformen mit einer erfindungsgemäßen Stopfenvorrichtung oder einer Stopfenvorrichtung gemäß einer oder mehreren der zuvor beschriebenen Ausführungsformen geschaffen wird. Weitere Ausgestaltungen sind in den abhängigen Ansprüchen beschrieben.

Es ist eine erste Substanz der zwei Substanzen in dem Hohlraum der Stopfenvorrichtung und eine zweite Substanz der zwei Substanzen in der Kammer des Grundkörpers angeordnet, wobei die Stopfenvorrichtung in distaler Richtung mit einer Kraft beaufschlagt wird, wobei die strömungstechnische Trennung der beiden Substanzen voneinander aufgehoben wird, wobei die Stopfenvorrichtung, insbesondere das Stopfenelement im Haltebereich, aufgrund der distalen Kraftbeaufschlagung zumindest teilweise so weit insbesondere radial verformt wird, dass das Verschlusselement aus der Halteposition heraus verlagert wird. Hierbei ist es ganz besonders vorteilhaft, dass die Beaufschlagung mit der Kraft und die daraus resultierende Verformung und/oder Verlagerung genügen, um die strömungstechnische Abdichtung zwischen der ersten und der zweiten Substanz aufzuheben, sodass die beiden Substanzen mit einfachen Mitteln und sicher zur Durchmischung gebracht werden können. Darüber hinaus ergeben sich die bereits zuvor bezüglich der Ausführungsbeispiele des Medikamentenbehälters und der Stopfenvorrichtung genannten Vorteile.

Insbesondere wird ein Verfahren zur Durchmischung zweier strömungstechnisch voneinander getrennt gelagerter Substanzen in einem Medikamentenbehälter insbesondere nach einer der zuvor beschriebenen Ausführungsbeispiele mit einer Stopfenvorrichtung insbesondere nach einem der zuvor beschriebenen Ausführungsbeispiele geschaffen, wobei eine erste Substanz der zwei Substanzen in dem Hohlraum der Stopfenvorrichtung und eine zweite Substanz der zwei Substanzen in der Kammer des Grundkörpers angeordnet ist, wobei die Stopfenvorrichtung in distaler Richtung mit einem Druck, insbesondere Fingerdruck, beaufschlagt wird, wobei die strömungstechnische Trennung der beiden Substanzen voneinander aufgehoben wird, wobei die Stopfenvorrichtung, insbesondere das Stopfenelement im Haltebereich, durch die distale Druckbeaufschlagung zumindest teilweise so weit insbesondere radial verformt wird, dass das Verschlusselement aus der Halteposition heraus verlagert wird. Hierbei ist es ganz besonders vorteilhaft, dass eine Verlagerung der Stopfenvorrichtung und/oder des Stopfenelements zur Öffnung des Hohlraums und damit zur Durchmischung der Substanzen nicht notwendig und insbesondere nicht vorgesehen ist. Eine Beaufschlagung mit Druck und die daraus resultierende Verformung genügen, um die strömungstechnische Abdichtung zwischen der ersten und der zweiten Substanz aufzuheben. Somit kann auch eine Durchmischung in einem Medikamentenbehälter, insbesondere Spritze oder Karpule, mit verschlossener Auslassöffnung, aber auch in einem Injektionsfläschchen oder einer Ampulle mit einer solchen Stopfenvorrichtung einfach durchgeführt werden, wobei große Druckschwankungen in den Behältern vermieden sind.

Vorzugsweise wird also das Stopfenelement an dessen proximalem Ende, insbesondere durch das Einwirken der Hervorspringung der Ausbringvorrichtung, auf die Stopfenvorrichtung verformt. Aufgrund der elastischen Ausbildung und der konusförmigen, insbesondere kegelförmigen Ausbildung der Hervorspringung wird der Haltebereich des Stopfenelements derart radial aufgeweitet, dass das von dem Haltemittel gehaltene Verschlusselement losgelassen wird.

Damit der Haltebereich ausreichend radial aufgeweitet werden kann, ist vorzugsweise vorgesehen, dass - bei bestimmungsgemäßer Einbaulage der Stopfenvorrichtung in dem Medikamentenbehälter - die Stopfenvorrichtung in dem Haltebereich nicht direkt an einer Innenwandung des Medikamentenbehälters anliegt. Vielmehr ist zwischen der Innenwandung des Medikamentenbehälters und einer Außenwandung des Stopfenelements im Haltebereich ein Freiraum vorgesehen, in welchen das Stopfenelement, insbesondere das Haltemittel, hineingedrängt wird, um das Verschlusselement freizugeben.

Wie bereits im Zusammenhang mit dem Medikamentenbehälter erläutert, verschließt das Verschlusselement in der Halteposition den Hohlraum, wobei der Hohlraum insbesondere gegenüber der Kammer des Medikamentenbehälters abgedichtet ist.

Insbesondere wird ein Verfahren zur Durchmischung zweier strömungstechnisch voneinander getrennt gelagerter Substanzen in einem Medikamentenbehälter insbesondere nach einem der zuvor beschriebenen Ausführungsbeispiele mit einer Stopfenvorrichtung insbesondere nach einem der zuvor beschriebenen Ausführungsbeispiele geschaffen, wobei eine erste Substanz der zwei Substanzen in dem Hohlraum der Stopfenvorrichtung und eine zweite Substanz der zwei Substanzen in der Kammer des Grundkörpers angeordnet ist, wobei die Stopfenvorrichtung in distaler Richtung verlagert wird, wobei die strömungstechnische Trennung der beiden Substanzen aufgehoben wird, wobei sich der Haltebereich während der distalen Verlagerung der Stopfenvorrichtung radial entspannt, wobei die Halterung des Verschlusselements in der Halteposition gelöst wird. Der Haltebereich der Stopfenvorrichtung, insbesondere des Stopfenelements, steht hier vorzugsweise unter einer radialen Vorspannung, indem der Haltebereich relativ zu einer entspannten Stellung, zusammengedrückt wird. In einem Entspannungsbereich, insbesondere dem zurückspringenden Bereich am distalen Ende der Einsteckhülse, welcher gegenüber der Einsteckhülse einen aufgeweiteten Innenradius aufweist, findet - nachdem das Stopfenelement in diesen hineinverlagert wurde - die Entspannung des Haltebereichs statt. Somit kann das Verschlusselement auf einfache Weise aus dem Haltebereich herausverlagert und dadurch der Hohlraum geöffnet werden. Unter einer Entspannung wird hier insbesondere eine aufweitende Verformung der vorgespannten Stopfenvorrichtung, insbesondere des Stopfenelements in dem Haltebereich verstanden.

Besonders bevorzugt ist vorgesehen, dass bei einem Verfahren nach einem der zuvor beschriebenen Ausführungsformen der Medikamentenbehälter nach dem Öffnen des Hohlraums, also nach dem Überführen der Stopfenvorrichtung in den zweiten Funktionszustand, geschüttelt wird. Somit ist die Durchmischung der beiden Substanzen verbessert.

Gemäß einer Weiterbildung der Erfindung ist vorgesehen, dass bei einem Verfahren nach einem der zuvor beschriebenen Ausführungsformen die Stopfenvorrichtung in dem Medikamentenbehälter zum vollständigen Ausbringen der ersten und/oder zweiten Substanz, insbesondere eines Medikaments, in eine distale Endstellung verlagert wird, wobei in der Endstellung das Verschlusselement vorzugsweise vollständig in dem Hohlraum aufgenommen wird, und/oder den Hohlraum vollständig von dem Verschlusselement ausgefüllt wird. Unter einer distalen Endstellung wird hier insbesondere eine Position des Stopfenelements am distalen Ende der Kammer des Grundkörpers verstanden, wobei das Stopfenelement insbesondere an der Auslassöffnung anliegt. Somit ist das Totvolumen bei der Verwendung eines Medikamentenbehälters der hier beschriebenen Art insbesondere bei der Durchführung der hier beschriebenen Verfahren minimal.

Die Beschreibungen des Verfahrens, der Stopfenvorrichtung und des Medikamentenbehälters sind komplementär zueinander zu verstehen. Insbesondere sind Merkmale der Stopfenvorrichtung und/oder des Medikamentenbehälters, die explizit oder implizit in Zusammenhang mit dem Verfahren beschrieben wurden, bevorzugt einzeln oder miteinander kombiniert Merkmale der Stopfenvorrichtung und/oder des Medikamentenbehälters. Bevorzugt sind die Stopfenvorrichtung und/oder der Medikamentenbehälter ausgebildet zur Durchführung wenigstens eines der in Zusammenhang mit dem Verfahren beschriebenen Verfahrensschritte. Verfahrensschritte, die explizit oder implizit in Zusammenhang mit der Stopfenvorrichtung und dem Medikamentenbehälter beschrieben wurden, sind bevorzugt einzeln oder in Kombination miteinander Schritte einer bevorzugten Ausführungsform des Verfahrens. Insbesondere ist im Rahmen des Verfahrens bevorzugt wenigstens ein Schritt vorgesehen, der sich aus wenigstens einem Merkmal der Stopfenvorrichtung und/oder des Medikamentenbehälters ergibt.

Insgesamt zeigt sich, dass mit der hier beschriebenen Stopfenvorrichtung, dem Medikamentenbehälter und dem Verfahren die Durchmischung von zwei Substanzen in einem Medikamentenbehälter verbessert ist. Auch ist die Handhabung, insbesondere während eines Herstellungsprozesses, verbessert, da die Befüllung des Hohlraums mit der ersten Substanz unabhängig von der Befüllung des Medikamentenbehälters mit der zweiten Substanz stattfinden kann. Insbesondere ist der Hohlraum der Stopfenvorrichtung auch bereits ohne einen Medikamentenbehälter strömungstechnisch abgedichtet, sodass eine darin gelagerte Substanz zum einen auch ohne Medikamentenbehälter bereits sicher gelagert ist, und zum anderen - bei bestimmungsgemäßer Einbaulage der Stopfenvorrichtung in dem Medikamentenbehälter - nicht in Kontakt mit einer silikonisierten Innenwandung des Medikamentenbehälters kommen kann. Somit ist insbesondere die aseptische Pulverabfüllung in einem Zweikammersystem mittels der hier beschriebenen Stopfenvorrichtung, dem hier beschriebenen Medikamentenbehälter und dem hier beschriebenen Verfahren auch im zuvor silikonisierten Medikamentenbehälter möglich, wobei die Sicherheit und die chemische Stabilität des abgefüllten Pulvers gewährleistet ist. Ferner ist bei der hier beschriebenen Stopfenvorrichtung, dem Medikamentenbehälter und den Verfahren die Haltbarkeit der Substanzen erhöht.

Die Erfindung wird im Folgenden anhand der Zeichnung näher erläutert. Dabei zeigen:
- Figur 1: einen Längsschnitt einer Stopfenvorrichtung gemäß einem nicht zur Erfindung gehörenden ersten Beispiel,
- Figur 2: einen Längsschnitt einer Stopfenvorrichtung gemäß einem nicht zur Erfindung gehörenden zweiten Beispiel,
- Figur 3: einen Längsschnitt eines Medikamentenbehälters gemäß einem nicht zur Erfindung gehörenden ersten Beispiel mit einer Stopfenvorrichtung gemäß dem nicht zur Erfindung gehörenden zweiten Beispiel,
- Figur 4: einen Längsschnitt einer Stopfenvorrichtung gemäß einem ersten Ausführungsbeispiel,
- Figur 5: einen Längsschnitt eines Medikamentenbehälters gemäß einem ersten Ausführungsbeispiel mit einer Stopfenvorrichtung gemäß dem ersten Ausführungsbeispiel in einem ersten Funktionszustand,
- Figur 6: einen Längsschnitt eines Medikamentenbehälters gemäß dem ersten Ausführungsbeispiel mit einer Stopfenvorrichtung gemäß dem ersten Ausführungsbeispiel in einem zweiten Funktionszustand, und
- Figur 7: einen Längsschnitt eines Medikamentenbehälters gemäß dem ersten Ausführungsbeispiel mit einer Stopfenvorrichtung gemäß dem ersten Ausführungsbeispiel in einer Endstellung der Stopfenvorrichtung in dem Medikamentenbehälter.

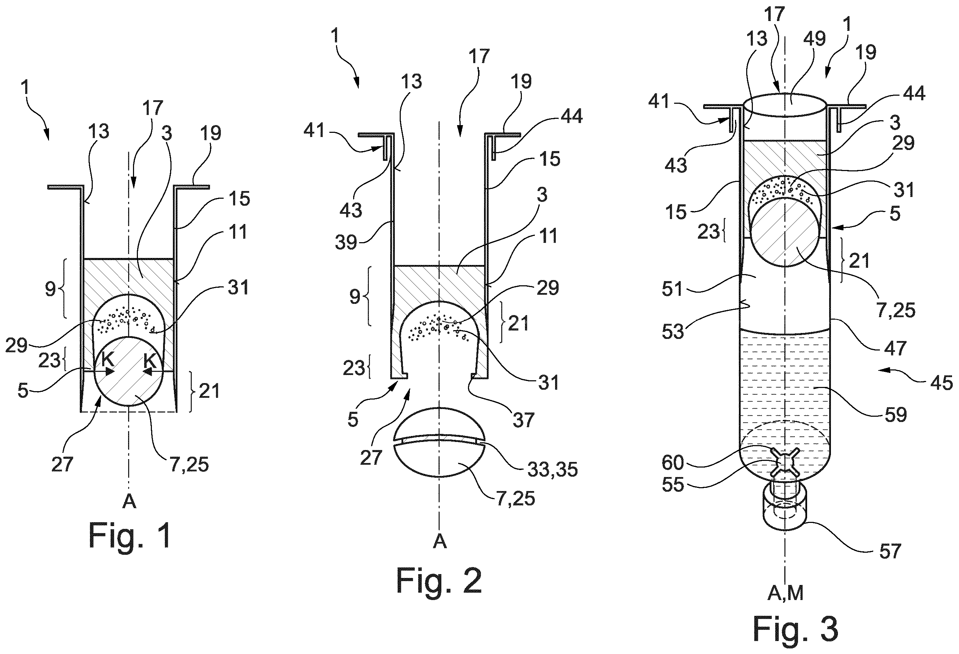

Figur 1 zeigt in einem nicht zur Erfindung gehörenden ersten Beispiel eine Stopfenvorrichtung 1 in einer Längsschnittdarstellung mit einem Stopfenelement 3, einem Haltemittel 5 und einem Verschlusselement 7. Das Stopfenelement 3 weist einen Dichtungsbereich 9 auf, in welchem eine Umfangsfläche 11 des Stopfenelements 3 dichtend ausgebildet ist. In dem Dichtungsbereich 9 liegt das Stopfenelement 3 insbesondere dicht an einer Innenseite 13 einer Einsteckhülse 15 an, sodass nach Montage der Stopfenvorrichtung 1 in einem hier nicht dargestellten Medikamentenbehälter der Medikamentenbehälter in einer axialen Richtung abgedichtet ist. Die hier dargestellte Stopfenvorrichtung 1 ist rotationssymmetrisch zu einer Rotationsachse A ausgebildet. In einer bestimmungsgemäßen Einbaulage in einem Medikamentenbehälter fällt die Rotationsachse mit einer Mittelachse des Medikamentenbehälters zusammen. Die Einsteckhülse 15 umhüllt also das Stopfenelement 3 in Umfangsrichtung um die Rotationsachse A und liegt dabei in radialer Richtung dicht an dem Stopfenelement 3 an.

Die Einsteckhülse 15 weist an ihrem in Figur 1 oberen, proximalen Ende eine eine proximale Öffnung 17 umlaufende Fingerplatte 19 auf. Mithilfe dieser Fingerplatte 19 kann die Stopfenvorrichtung 1 und/oder ein Medikamentenbehälter gehalten werden, insbesondere um das Stopfenelement 3 mit einer Kraft in distaler Richtung zu beaufschlagen. An ihrem in Figur 1 unteren, distalen Ende weist die Einsteckhülse 15 einen zurückspringenden Bereich 21 auf, wobei die Enden der Einsteckhülse 15 in dem zurückspringenden Bereich 21 in dem hier dargestellten Längsschnitt durch die Stopfenvorrichtung 1 keilförmig, insbesondere als Ablaufschräge ausgebildet sind.

Das Haltemittel 5 ist hier in einem Haltebereich 23 der Stopfenvorrichtung 1 vorgesehen, wobei der Haltebereich 23 von einem in Figur 1 unteren axialen Abschnitt des Stopfenelements 3 ausgebildet ist. In diesem Haltebereich 23 ist das Stopfenelement 3, welches elastisch ausgebildet ist, gegenüber einer entspannten Stellung zusammengedrückt, sodass das Haltemittel 5 radial vorgespannt ist.

Das Stopfenelement 3 ist entlang der Rotationsachse - in Figur 1 also nach oben und/oder unten - in der Einsteckhülse 15 verlagerbar angeordnet. Nach Einführen der Stopfenvorrichtung 1 in den hier nicht dargestellten Medikamentenbehälter ist das Stopfenelement 3 insbesondere nach unten, in eine distale Richtung, verlagerbar, wobei der Medikamentenbehälter in jeder distalen Verlagerungsstellung axial mittels der Stopfenvorrichtung 1, insbesondere des Stopfenelements 3 und der Einsteckhülse 15, abgedichtet ist.

Die Stopfeneinrichtung 1 ist in Figur 1 in einem ersten Funktionszustand dargestellt, wobei das Verschlusselement 7 mittels des Haltemittels 5 ortsfest zu dem Stopfenelement 3 in einer Halteposition lösbar gehalten ist. Das Verschlusselement 7 wird in dem hier dargestellten nicht zur Erfindung gehörenden Beispiel insbesondere durch Kraftschluss gehalten, wobei das Haltemittel 5 insbesondere aufgrund der rotationssymmetrischen Ausbildung von allen Seiten in dem Haltebereich 23 das Verschlusselement 7 mit einer Kraft K nach radial innen beaufschlagt. Das Verschlusselement 7, welches hier insbesondere als Kugel 25 ausgebildet ist, ist also in einer unteren, distalen Öffnung 27 des Stopfenelements 3 eingeklemmt.

Die untere, distale Öffnung 27 des Stopfenelements 3 ist durch das Verschlusselement 7 strömungstechnisch dicht verschlossen. Zwischen dem Verschlusselement 7 und dem Stopfenelement 3 ist dadurch ein Hohlraum 29 ausgebildet, in welchem eine erste Substanz 31, hier insbesondere eine Pulverarznei, angeordnet ist. Somit ist hier ein strömungstechnisch dichter Hohlraum 29 geschaffen, in welchem die erste Substanz 31 sicher gelagert werden kann, wobei ein Medikamentenbehälter nicht erforderlich ist, um die strömungstechnische Abdichtung zu realisieren.

Wie in Figur 1 erkennbar ist, ragt das Verschlusselement 7 nicht über ein unteres, distales Ende der Einsteckhülse 15 hinaus, sodass das Verschlusselement 7 gegen eine ungewollte Verlagerung, insbesondere in Richtung des Hohlraums 29, oder eine anderweitig störende Verlagerung, welche geeignet wäre, die Dichtigkeit des Hohlraums 29 zu gefährden, geschützt ist. Somit kann die erste Substanz 31 in dieser Stopfenvorrichtung 1 sicher gelagert werden und zu einem beliebigen, späteren Zeitpunkt in einem Medikamentenbehälter montiert werden.

Figur 2 zeigt eine Stopfenvorrichtung 1 in einer seitlichen Schnittdarstellung gemäß einem nicht zur Erfindung gehörenden zweiten Beispiel. Die Stopfenvorrichtung 1 ist hier insbesondere in einem zweiten Funktionszustand dargestellt, wobei das Verschlusselement 7 nicht von dem Haltemittel 5 in der Halteposition gehalten ist. Gleiche und funktionsgleiche Teile sind mit gleichen Bezugszeichen versehen, sodass insofern auf die vorangegangene Figurenbeschreibung verwiesen wird.

Anders als in dem in Figur 1 dargestellten nicht zur Erfindung gehörenden ersten Beispiel ist hier in Figur 2 der Hohlraum 29 offen. Das Verschlusselement 7 ist nicht von dem Haltemittel 5 in der Halteposition gehalten. Die in dem Hohlraum 29 angeordnete erste Substanz 31 kann somit aus dem Hohlraum 29 entweichen.

Das Stopfenelement 3 weist hier - wie auch in Figur 1 - den Dichtungsbereich 9 und den Haltebereich 23 auf. Auch in Figur 2 ist vorgesehen, dass der Haltebereich 23 zumindest in dem hier nicht dargestellten ersten Funktionszustand durch Zusammendrücken nach radial innen vorgespannt ist, sodass das Verschlusselement 7 in der unteren, distalen Öffnung 27 des Stopfenelements 3 eingeklemmt ist. Durch Verlagern des Stopfenelements 3 in den hier darstellten, zweiten Funktionszustand ist ein Teil des Stopfenelements 3, insbesondere nämlich der Haltebereich 23, aus der Einsteckhülse 15 herausverlagert, sodass sich das Stopfenelement 3 in dem Haltebereich 23 und damit auch das Haltemittel 5 nach radial außen entspannen kann. Aufgrund der radialen Entspannung lassen die Haltekräfte K auf das Verschlusselement 7 nach und das Verschlusselement 7 ist nicht länger in der Halteposition gehalten.

Zusätzlich zu dem oben beschriebenen Haltemechanismus, welcher genauso bei dem nicht zur Erfindung gehörenden ersten Beispiel, welches in Figur 1 dargestellt ist, realisiert ist, ist in Figur 2 eine Rastaufnahme 33 an dem Verschlusselement 7 vorgesehen. Diese Rastaufnahme 33 wird hier insbesondere durch eine äquatorial umlaufende Rastnut 35 gebildet. Korrespondierend zu dieser Rastnut 35 ist vorgesehen, dass das Haltemittel 5 einen die untere, distale Öffnung 27 umlaufenden Rastvorsprung 37 aufweist, welcher - in dem ersten Funktionszustand - formschlüssig in die Rastnut 35 eingreift. Somit ist eine zusätzliche Sicherung zum Halten des Verschlusselements 7 in der Halteposition geschaffen.

Zum Lösen des Verschlusselements 7 aus der Halteposition ist es in dem in Figur 2 dargestellten nicht zur Erfindung gehörenden zweiten Beispiel der Stopfenvorrichtung 1 erforderlich, dass der Haltebereich 23 in dem ersten Funktionszustand zumindest so weit zusammengedrückt ist, wie der Rastvorsprung 37 lang ist. Unter der Länge des Rastvorsprungs wird hier insbesondere die Erstreckung des Rastvorsprungs in radialer Richtung verstanden. Dadurch ist gewährleistet, dass die aus der Vorspannung resultierende Rückstellkraft das Haltemittel 5, insbesondere den Rastvorsprung 37, zumindest soweit radial aufweitet, dass der Rastvorsprung 37 nicht mehr in Eingriff mit der Rastnut 35 ist. Ferner ist es bevorzugt vorgesehen, dass eine Dicke einer Wandung 39 der Einsteckhülse 15 genauso dick ist wie oder dicker ist als die Länge des Rastvorsprungs 37. Dadurch ist sichergestellt, dass der Haltebereich 23 - bei bestimmungsgemäßer Einbaulage in einem Medikamentenbehälter mit einem zylinderförmigen Innenraum - weit genug entspannen kann, um den Formschluss zwischen Rastnut 35 und Rastvorsprung 37 aufzulösen.

In Figur 2 ist weiterhin erkennbar, dass die Einsteckhülse 15 eine Befestigungsstruktur 41 zur Befestigung der Einsteckhülse 15 an einem Medikamentenbehälter aufweist. Diese Befestigungsstruktur 41 weist eine Aufnahme 43 für einen Medikamentenbehälter auf, in welchem der Medikamentenbehälter mit seinem proximalen Ende rastend und/oder klemmend aufnehmbar ist. Somit ist die Stopfenvorrichtung 1 auf einfache Weise an dem Medikamentenbehälter befestigbar, insbesondere anklipsbar. Alternativ oder zusätzlich kann die Stopfenvorrichtung 1 vorzugsweise mittels der Befestigungsstruktur 41 mit dem Medikamentenbehälter stoffschlüssig verbunden, insbesondere an den Medikamentenbehälter aufgeschweißt, angeschweißt und/oder geklebt sein, wodurch die Sterilität in dem Innenraum des Medikamentenbehälters besonders zuverlässig gewährleistet ist.

Die in Figur 2 dargestellte Aufnahme 43 erstreckt sich in Richtung der Mittelachse lediglich über einen kleinen Abschnitt der Einsteckhülse 15. Vorzugsweise erstreckt sich die Aufnahme 43 jedoch in einem hier nicht dargestellten, alternativen Ausführungsbeispiel über eine gesamte axiale Erstreckung der Einsteckhülse 15. Dies ist in dem hier nicht dargestellten, alternativen Ausführungsbeispiel vorzugsweise dadurch realisiert, dass eine äußere Wand 44 der Aufnahme 43 sich insbesondere parallel zu der Innenseite 13 der Einsteckhülse 15 über die gesamte axiale Erstreckung der Einsteckhülse 15 erstreckt. Besonders bevorzugt erstreckt sich die äußere Wand 44 der Aufnahme 43 - in dem ersten Funktionszustand - über eine gesamte axiale Erstreckung des Verschlusselements 7, vorzugsweise - in distaler Richtung - darüber hinaus. Somit kann eine insbesondere zusätzliche Abdichtung zwischen der äußeren Wand 44 der Aufnahme 43 und einer Außenwandung des Medikamentenbehälters bewirkt werden, sodass die Sterilität und die Sicherheit des Medikamentenbehälters erhöht ist.

Figur 3 zeigt einen Medikamentenbehälter 45 mit einer Stopfenvorrichtung 1 gemäß dem in Figur 2 beschriebenen nicht zur Erfindung gehörenden Beispiel. Der Medikamentenbehälter 45 weist einen zylindrischen Grundkörper 47 und ein proximales Ende 49 auf. Dabei wird eine Kammer 51 des Medikamentenbehälters 45, welche zur Aufnahme einer medizinischen Substanz eingerichtet ist, von einer insbesondere silikonisierten Innenwandung 53 des Grundkörpers 47, der Stopfenvorrichtung 1 und - in distaler Richtung - von einer distalen Auslassöffnung 55 begrenzt. Die distale Auslassöffnung 55 ist hier in Figur 3 mittels einer Verschlusskappe 57 verschlossen. In der Kammer 51 ist eine zweite Substanz 59, insbesondere ein Lösungsmittel für die Pulverarznei, angeordnet.

Indem die Stopfenvorrichtung 1 bestimmungsgemäß in dem Medikamentenbehälter 45 eingebaut ist, fällt die Rotationsachse A der Stopfenvorrichtung 1 mit einer Mittelachse M des Medikamentenbehälters 45 zusammen, wobei das Stopfenelement 3 entlang der Mittelachse A insbesondere in distale Richtung verlagerbar ist.

Der Übersichtlichkeit halber sind die in Figur 2 dargestellten Raststrukturen, nämlich die Rastnut 35 und der Rastvorsprung 37, hier in Figur 3 nicht dargestellt, aber dennoch vorgesehen. Auch die Haltekräfte K sind hier vorgesehen, der Übersichtlichkeit halber aber nicht eingezeichnet.

Die Stopfenvorrichtung 1 befindet sich hier in dem ersten Funktionszustand, sodass der Hohlraum 29 dicht verschlossen ist. Die in dem Hohlraum 29 angeordnete erste Substanz 31 ist damit strömungstechnisch von der Kammer 51, der darin angeordneten zweiten Substanz 59 und auch der insbesondere silikonisierten Innenwandung 53 des Medikamentenbehälters 45 abgedichtet. Somit ist eine Reaktion zwischen dem Silikon und der ersten Substanz 31 verhindert.

Die Auslassöffnung 55 des Grundkörpers 47 weist auf ihrer der Kammer 51 zugewandten Seite eine Geometrie auf, welche von einer Form des Verschlusselements 7 abweicht. Insbesondere ist diese Geometrie hier durch einen unrunden Querschnitt 60 realisiert, sodass das mit Ausnahme der Rastnut 35 kugelförmig ausgebildete Verschlusselement 7 sich nicht dichtend auf die Auslassöffnung 55 legen kann und die Auslassöffnung 55 somit stets an ihrer der Kammer 51 zugewandten Seite von innen unverschlossen bleibt. Vorzugsweise ist der Querschnitt 60 zumindest teilweise kreuzförmig ausgebildet.

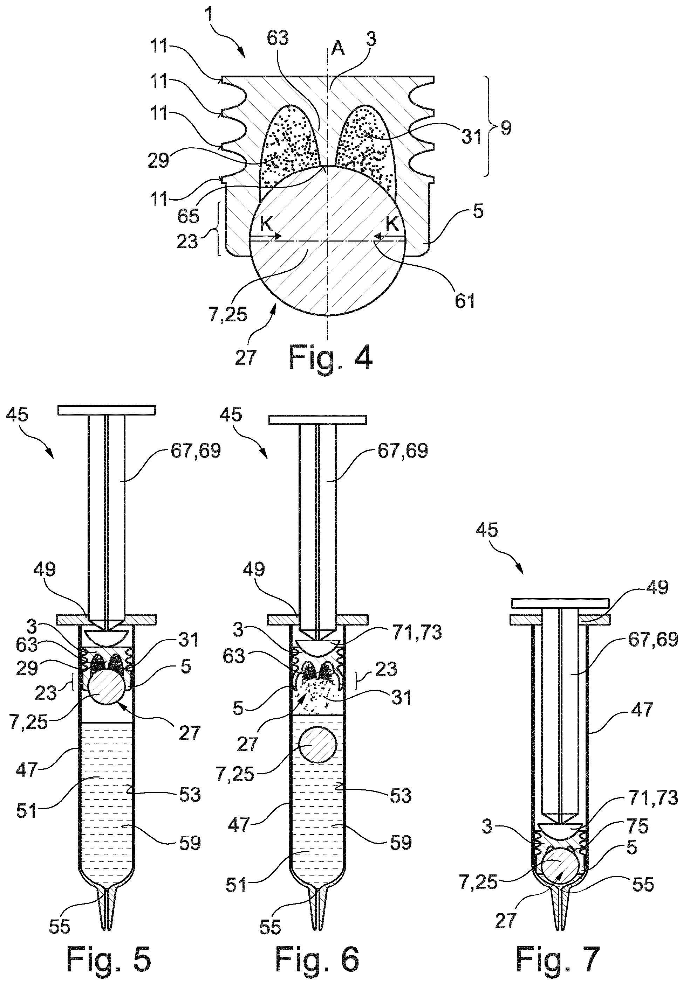

Figur 4 zeigt eine Stopfenvorrichtung 1 in einer seitlichen Schnittdarstellung gemäß einem ersten Ausführungsbeispiel. Gleiche und funktionsgleiche Teile sind mit gleichen Bezugszeichen versehen, sodass insofern auf die vorangehende Figurenbeschreibung verwiesen wird.

Die hier dargestellte Stopfenvorrichtung 1 weist im Unterschied zu dem ersten und dem zweiten nicht zur Erfindung gehörenden Beispiel der Stopfenvorrichtung 1 keine Einsteckhülse 19 auf.

Wie auch die zuvor beschriebenen nicht zur Erfindung gehörenden Beispiele der Stopfenvorrichtung 1 weist das erste Ausführungsbeispiel der Stopfenvorrichtung 1, welches in Figur 4 dargestellt ist, ein Stopfenelement 3 auf, welches ausgebildet ist, einen Medikamentenbehälter 45 in einer axialen Richtung abzudichten. Die hier dargestellte Stopfenvorrichtung 1 ist insbesondere komplett in einen Medikamentenbehälter 45 einführbar, wobei das Stopfenelement 3, hier insbesondere die komplette Stopfenvorrichtung 1 entlang der Mittelachse 11 in dem Medikamentenbehälter 45 verlagerbar ausgebildet ist.

Die Stopfenvorrichtung 1 weist einen Haltebereich 23 mit einem Haltemittel 5 und einen Dichtungsbereich 9 auf, welcher einer Mehrzahl von Umfangsflächen 11 des Stopfenelements 3 aufweist. Vorzugsweise ist jede der Mehrzahl von Umfangsflächen 11 ausgebildet, um - bei bestimmungsgemäßer Einbaulage der Stopfenvorrichtung 1 in dem Medikamentenbehälter 45 - dichtend an einer Innenwandung 53 des Medikamentenbehälters 45 anzuliegen.

Das Haltemittel 5 hält ein Verschlusselement 7 in einer Halteposition. Dabei wird das Verschlusselement 7, welches als Kugel 25 ausgebildet ist, an dem Stopfenelement 3 unmittelbar gehalten.

Zwischen dem Verschlusselement 7 und dem Stopfenelement 3 ist ein Hohlraum 29 ausgebildet, welcher in Figur 4 aufgrund der seitlichen Schnittdarstellung zweigeteilt erscheint, jedoch aufgrund der Rotationssymmetrie um die Rotationsachse A ein zusammenhängender, ringförmiger Hohlraum 29 ist.

Das Haltemittel 5 hält das Verschlusselement 7 in der Halteposition insbesondere kraftschlüssig, indem von dem Haltebereich 23 des Stopfenelements 3 ausgehend Haltekräfte K nach radial innen wirken und dadurch das Verschlusselement 7 in einer unteren, distalen Öffnung 27 einklemmen. Außerdem ist somit in dem Haltebereich 23 die dichte Anlage des Stopfenelements 3 an dem Verschlusselement 7 und damit die Abdichtung des Hohlraums 29 sichergestellt.

Zusätzlich zu dieser Klemmwirkung des Haltemittels 5 ist bei dem in Figur 4 dargestellten Ausführungsbeispiel der Stopfenvorrichtung 1 vorgesehen, dass sich der Haltebereich 23 des Stopfenelements 3 - in dem hier dargestellten ersten Funktionszustand - zumindest teilweise über einen Äquator 61 der Kugel 25 hinaus in distale Richtung erstreckt, wobei der Haltebereich 23 an dem Verschlusselement 7 anliegt, sodass das Verschlusselement 7 von dem Haltemittel 5 in distaler Richtung hintergriffen ist. Somit ist das Verschlusselement auch formschlüssig gehalten.

Unter einem Äquator 61wird hier insbesondere eine geschlossene Umfangslinie um das Verschlusselement 7 verstanden, welche eine Fläche einschließt, die auf der Rotationsachse A senkrecht steht und einen Radius aufweist, der einem Radius des Verschlusselements 7 entspricht, wobei der Radius bezüglich der Längserstreckung der Mittelachse zumindest lokal, vorzugsweise global maximal ist.

Das Stopfenelement 3 weist - anders als die beiden zuvor beschriebenen nicht zur Erfindung gehörenden Beispiele in den Figuren 1, 2 und 3 - auf seiner dem Verschlusselement 7 zugewandten Seite einen zumindest teilweise in Richtung des Verschlusselements 7 ausgerichteten Ausbringvorsprung 63 auf. Dieser Ausbringvorsprung 63 springt konusähnlich - insbesondere kegelstumpfförmig - von dem Stopfenelement 3 hervor und stößt mit einer vorderen, in Figur 4 unteren Stirnfläche 65 des Ausbringvorsprungs 63 gegen das Verschlusselement 7.

Somit findet sich die Stopfenvorrichtung 1 hier in einem Auswurfszustand, in welchem bereits eine geringe Verformung, insbesondere ein axiales Einwirken des Ausbringvorsprungs 63 auf das Verschlusselement 7 einen Auswurf des Verschlusselements 7 aus der Halteposition bewirken kann.

Die Figuren 5 bis 7 zeigen einen Medikamentenbehälter 45 mit einer Stopfenvorrichtung 1 gemäß dem in Figur 4 beschriebenen Ausführungsbeispiel, wobei die Stopfenvorrichtung 1 - in verschiedenen Funktionszuständen - in bestimmungsgemäßer Einbaulage in dem Medikamentenbehälter angeordnet ist. Anhand dieser Figuren wird im Folgenden ein Verfahren zur Durchmischung zweier strömungstechnisch voneinander gelagerten Substanzen, nämlich einer ersten Substanz 31 und einer zweiten Substanz 59 in dem Medikamentenbehälter 45 erläutert.

In Figur 5 ist die erste Substanz 31 in dem Hohlraum 29 der Stopfenvorrichtung strömungstechnisch dicht durch das Stopfenelement 3 und das Verschlusselement 7 eingeschlossen. Die in Figur 5 dargestellte Stopfenvorrichtung 1 befindet sich also in dem ersten Funktionszustand.

Um das Verschlusselement 7 aus der Halteposition zu lösen und damit den Hohlraum 29 zu öffnen, wird - wie in Figur 6 dargestellt - das Stopfenelement 3 in distaler Richtung mit einem Druck beaufschlagt wird. Durch die daraus resultierende Verformung insbesondere in dem proximalen Bereich des Stopfenelements 3 wird ein distales Ende und damit auch die untere, distale Öffnung 27 des Stopfenelements 3 nach radial außen aufgespreizt, sodass zum einen der durch das Haltemittel 5 bewirkte Kraftschluss und zum anderen der durch das Hintergreifen über den Äquator 61 des Verschlusselements 7 hinaus bewirkte Formschluss aufgehoben wird. Infolgedessen ist das Verschlusselement 7 nicht mehr in der Halteposition gehalten und wird aufgrund der Gravitation und/oder einer Schüttelbewegung aus der Halteposition herausverlagert. Der Hohlraum 29 ist nun also geöffnet, und die erste Substanz 31 vermischt sich aufgrund der strömungstechnischen Öffnung des Hohlraums 29 mit der zweiten Substanz 59. Das Verschlusselement 7 ist hier insbesondere als Mischkörper ausgebildet, welcher beim Schütteln des Medikamentenbehälters 45 zu zusätzlichen Turbulenzen in der Kammer 51 des Medikamentenbehälters 45 führt, welche die Durchmischung beschleunigen und/oder verbessern.

Hervorzuheben ist hier, dass eine Verlagerung des Stopfenelements 3 nicht zwingend notwendig ist, um den Hohlraum 29 zu öffnen. Vielmehr genügt allein die Beaufschlagung mit dem Druck in distaler Richtung, um eine geeignete Verformung des Stopfenelements 3 herbeizuführen.

Die Verformung des Stopfenelements 3 wird hier insbesondere mittels einer Ausbringvorrichtung 67, welche als Kolbenstange 69 ausgebildet ist, bewirkt. An einem distalen Ende 71 der Ausbringvorrichtung 67 ist eine konvexe, kugelabschnittsförmige Hervorspringung 73 vorgesehen. Diese Hervorspringung 73 ist rotationssymmetrisch zu einer Mittelachse M des Medikamentenbehälters 45 ausgebildet. Zentral auf der Mittelachse M ist die Hervorspringung 73 dabei maximal. Somit ist bei Druckbeaufschlagung des Stopfenelements 3 mittels der Ausbringvorrichtung 67 die Verformung in einem zentralen Bereich in unmittelbarer Nähe zu der Mittelachse M am größten, sodass das Haltemittel 5 im Haltebereich 23 hebelartig in zumindest zwei vorzugsweise einander gegenüberliegende, radiale Richtungen, besonders bevorzugt allseitig in jede radiale Richtung, aufgespreizt wird.

Figur 7 zeigt den Medikamentenbehälter 45 in einer Endstellung, wobei das Verschlusselement 7 nahezu vollständig in dem Hohlraum 29 aufgenommen ist, sodass der Hohlraum 29 seinerseits nahezu vollständig von dem Verschlusselement 7 ausgefüllt wird. Somit ist eine Restmenge der ersten Substanz und/oder der zweiten Substanz in der Kammer 51 des Medikamentenbehälters 45 minimal. Zu diesem Zweck ist das Stopfenelement 3, insbesondere der Ausbringvorsprung 63 elastisch ausgebildet, sodass sich der Ausbringvorsprung 63 in der Endstellung verformt, sodass ein Restvolumen 75 des Hohlraums 29 minimal ist. Dennoch weist der Ausbringvorsprung 63 eine ausreichende Festigkeit auf, um - wie in den Figuren 5 und 6 dargestellt - das Verschlusselement 7 entgegen der Haltekräfte K aus der Halteposition heraus zu verlagern, wobei die Haltekräfte K durch eine Verformung des Stopfenelements 3 vorzugsweise reduziert werden.

Insgesamt zeigt sich, dass mittels einer solchen Stopfenvorrichtung 1 und eines solchen Medikamentenbehälters 45 eine erste, insbesondere instabile Substanz 31 sicher und reaktionsfrei vorabgefüllt in der Stopfenvorrichtung 1 strömungstechnisch dicht gelagert ist, und, insbesondere mittels eines solchen Verfahrens, in dem Medikamentenbehälter 45 zur Durchmischung mit einer zweiten Substanz 59 gebracht werden kann, wobei auch hier das Risiko einer Reaktion, insbesondere einer ungewollten Reaktion mit einer silikonisierten Innenwandung 53 des Medikamentenbehälters 45, minimal ist. Außerdem ist die Handhabung gegenüber herkömmlichen Stopfenvorrichtungen 1 und/oder Medikamentenbehältern 45 verbessert, da die Stopfenvorrichtung 1 unabhängig von dem Medikamentenbehälter 45 mit der ersten Substanz 31 vorabgefüllt und gegebenenfalls verpackt werden kann, ohne dass ein Medikamentenbehälter 45 notwendig ist. Außerdem ist in einem solchen Medikamentenbehälter 45 das Totvolumen, also eine Restmenge der ersten Substanz 31 und/oder der zweiten Substanz 59 in der Kammer 51 minimiert.

## Patentansprüche

1. Stopfenvorrichtung (1) zur Abdichtung eines Medikamentenbehälters (45), mit einem Stopfenelement (3), wobei die Stopfenvorrichtung (1) ausgebildet ist, den Medikamentenbehälter (45) in einer axialen Richtung einer Mittelachse (M) des Medikamentenbehälters (45) abzudichten, wobei die Stopfenvorrichtung (1) in den Medikamentenbehälter (45) zumindest teilweise einführbar und das Stopfenelement (3) bei bestimmungsgemäßer Einbaulage in dem Medikamentenbehälter (45) entlang der Mittelachse (M) des Medikamentenbehälters (45) verlagerbar ausgebildet ist, wobei die Stopfenvorrichtung (1) einen Haltebereich (23) mit zumindest einem Haltemittel (5) aufweist, wobei ein Verschlusselement (7) - in einem ersten Funktionszustand der Stopfenvorrichtung (1) - mittels des Haltemittels (5) ortsfest zu dem Stopfenelement (3), insbesondere an dem Stopfenelement (3), in einer Halteposition lösbar derart gehalten ist, dass zwischen dem Verschlusselement (7) einerseits und dem Stopfenelement (3) andererseits ein Hohlraum (29) ausgebildet ist, wobei das Verschlusselement (7) als kugelförmiger Mischkörper für den Medikamentenbehälter (45) ausgebildet ist, **dadurch gekennzeichnet, dass** das Stopfenelement (3) - in dem ersten Funktionszustand - auf seiner dem Verschlusselement (7) zugewandten Seite einen zumindest teilweise in Richtung des Verschlusselements (7) ausgerichteten Ausbringvorsprung (63) aufweist.

2. Stopfenvorrichtung (1) nach Anspruch 1, **dadurch gekennzeichnet, dass** der Hohlraum (29) im ersten Funktionszustand strömungstechnisch gegen eine Wandung, insbesondere Innenwandung (53), des Medikamentenbehälters (45) abgedichtet ist.

3. Stopfenvorrichtung (1) nach einem der vorhergehenden Ansprüche, **dadurch gekennzeichnet, dass** der Hohlraum (29) in einem zweiten Funktionszustand offen ist, wobei das Verschlusselement (7) nicht von dem Haltemittel (5) in der Halteposition gehalten ist.

4. Stopfenvorrichtung (1) nach einem der vorhergehenden Ansprüche, **dadurch gekennzeichnet, dass** das Verschlusselement (7) - im zweiten Funktionszustand - frei beweglich in dem Medikamentenbehälter (45) angeordnet ist.

5. Stopfenvorrichtung (1) nach einem der vorhergehenden Ansprüche, **dadurch gekennzeichnet, dass** der Ausbringvorsprung (63) - zumindest in einem Auswurfszustand der Stopfenvorrichtung (1) - mit einer dem Verschlusselement (7) zugewandten Seite und/oder Spitze an dem Verschlusselement (7) anliegt.

6. Stopfenvorrichtung (1) nach einem der vorhergehenden Ansprüche, **dadurch gekennzeichnet, dass** das Verschlusselement (7) zumindest ein erstes Rastmittel aufweist, wobei das Haltemittel (23) der Stopfenvorrichtung (1) zumindest ein zu dem ersten Rastmittel korrespondierend ausgebildetes zweites Rastmittel aufweist, welches in dem Haltebereich (23) an einer dem Verschlusselement (7) zugewandten Seite des Stopfenelements (3) angeordnet ist, wobei das erste Rastmittel und das zweite Rastmittel - in dem ersten Funktionszustand - rastend ineinandergreifen.

7. Stopfenvorrichtung (1) nach einem der vorhergehenden Ansprüche, **dadurch gekennzeichnet, dass** die Stopfenvorrichtung (1) eine das Stopfenelement (3) umgreifende Einsteckhülse (15) aufweist, wobei bevorzugt eine Außenseite einer Hülsenwand zur dichten Anlage an einer Innenwandung (53) des Medikamentenbehälters (45) ausgebildet ist.

8. Stopfenvorrichtung (1) nach Anspruch 7, **dadurch gekennzeichnet, dass** die Einsteckhülse (15) als Fingerauflage ausgebildet ist und an einem proximalen Ende zumindest eine radial abstehende Fingerplatte (19) aufweist, welche von einem Finger hintergreifbar ausgebildet ist.

9. Stopfenvorrichtung (1) nach einem der Ansprüche 7 oder 8, **dadurch gekennzeichnet, dass** die Einsteckhülse (15) eine Befestigungsstruktur (41) zur Befestigung der Einsteckhülse (15) an dem Medikamentenbehälter (45) aufweist, wobei die Befestigungsstruktur (41) vorzugsweise eine Aufnahme (43) für den Medikamentenbehälter (45) aufweist, in welcher der Medikamentenbehälter (45) mit seinem proximalen Ende (49) rastend und/oder klemmend aufnehmbar ist.

10. Medikamentenbehälter mit einem Grundkörper (47) und einer Stopfenvorrichtung (1) nach einem der vorhergehenden Ansprüche, wobei die Stopfenvorrichtung (1) bereichsweise derart in den Medikamentenbehälter (45) hinein verlagert angeordnet ist, dass das Stopfenelement (3) ein proximales Ende (49) des Medikamentenbehälters (45) gegenüber einer Kammer (51) des Grundkörpers (47) abdichtet, wobei die Kammer (51) in radialer Richtung von einer Innenwandung (53) des Grundkörpers (47) und in distaler Richtung von einer Auslassöffnung (55) des Grundkörpers (47) und/oder einer Verschlusskappe (57) begrenzt ist.

11. Medikamentenbehälter nach Anspruch 10, **dadurch gekennzeichnet, dass** die Auslassöffnung (55) des Grundkörpers (47) zumindest auf ihrer der Kammer (51) zugewandten Seite eine Geometrie, insbesondere einen kreuzförmigen Querschnitt (60), aufweist, welche von einer der Auslassöffnung zugewandten Seite des Verschlusselements (7) derart abweicht, dass ein strömungstechnisch dichter Verschluss der Auslassöffnung (55) durch das Verschlusselement (7) verhindert ist.

12. Medikamentenbehälter nach Anspruch 10 oder 11, **gekennzeichnet durch** eine Ausbringvorrichtung (67), wobei an einem distalen Ende (71) der Ausbringvorrichtung (67) eine Hervorspringung (73) vorgesehen ist.

13. Medikamentenbehälter nach Anspruch 12, **dadurch gekennzeichnet, dass** das Stopfenelement (3) elastisch ausgebildet ist, wobei es mittels der Hervorspringung (73) der Ausbringvorrichtung (67) durch insbesondere manuelle Druckausübung in distaler Richtung auf die Ausbringvorrichtung (67) verformbar ist, wobei der Ausbringvorsprung (63) des Stopfenelements (3) eine Festigkeit in axialer Richtung aufweist, die größer ist als es dem zur Ausbringung des Verschlusselements (7) erforderlichen Druck entspricht.

14. Verfahren zur Durchmischung zweier strömungstechnisch voneinander getrennt gelagerten Substanzen (31,59) in einem Medikamentenbehälter (45) nach einem der Ansprüche 10 bis 13 mit einer Stopfenvorrichtung (1) nach einem der Ansprüche 1 bis 9, wobei eine erste Substanz (31) der zwei Substanzen (31,59) in dem Hohlraum (29) der Stopfenvorrichtung (1) und eine zweite Substanz (59) der zwei Substanzen (31,59) in der Kammer (51) des Grundkörpers (47) angeordnet ist, wobei das Stopfenelement (3) in distaler Richtung mit einer Kraft beaufschlagt wird, wobei die strömungstechnische Trennung der beiden Substanzen (31,59) aufgehoben wird, **dadurch gekennzeichnet, dass** die Stopfenvorrichtung (1), insbesondere das Stopfenelement (3), aufgrund der distalen Kraftbeaufschlagung zumindest teilweise so weit verformt wird, dass das Verschlusselement (7) aus der Halteposition heraus verlagert wird.

15. Verfahren nach Anspruch 14, **dadurch gekennzeichnet, dass** das Stopfenelement (3) in distaler Richtung verlagert wird, wobei die strömungstechnische Trennung der beiden Substanzen (31,59) aufgehoben wird, wobei sich der Haltebereich (23) während der distalen Verlagerung der Stopfenvorrichtung (1) radial entspannt, und wobei die Halterung des Verschlusselements (7) in der Halteposition gelöst wird.

16. Verfahren nach Anspruch 14 oder 15, **dadurch gekennzeichnet, dass** der Medikamentenbehälter (45) nach dem Öffnen des Hohlraums (29) geschüttelt wird.

17. Verfahren nach einem der Ansprüche 14 bis 16, **dadurch gekennzeichnet, dass** die Stopfenvorrichtung (1) zum vollständigen Ausbringen der zwei Substanzen (31,59) in dem Medikamentenbehälter (45) in eine Endstellung verlagert wird, wobei das Verschlusselement (7) in der Endstellung in dem Hohlraum (29) aufgenommen wird, und wobei der Hohlraum (29) vollständig von dem Verschlusselement (7) ausgefüllt wird.

## Claims

1. Stopper device (1) for sealing off a medicament container (45), with a stopper element (3), wherein the stopper device (1) is embodied to seal off the medicament container (45) in an axial direction of a center axis (M) of the medicament container (45), wherein the stopper device (1) is at least partially insertable into the medicament container (45) and in the intended installation position in the medicament container (45) the stopper element (3) is displaceable along the center axis (M) of the medicament container (45), wherein the stopper device (1) has a retention region (23) with at least one retention means (5), wherein - in a first functional state of the stopper device (1) - a closure element (7) is releasably retained in a fixed position relative to the stopper element (3) by means of the retention means (5), in particular on the stopper element (3), in such a way that a cavity (29) is formed between the closure element (7) on the one hand and the stopper element (3) on the other hand, wherein the closure element (7) is designed as a spherical mixing body for the medicament container (45), **characterized in that** - in the first functional state - the stopper element (3) has a discharge projection (63) on its side facing the closure element (7) and the discharge projection (63) is at least partially oriented in the direction of the closure element (7).

2. Stopper device (1) according to claim 1, **characterized in that** in the first functional state the cavity (29) is fluidically sealed off with respect to a wall, in particular an inner wall (53), of the medicament container (45).

3. Stopper device (1) according to one of the preceding claims, **characterized in that** in a second functional state the cavity (29) is open, wherein the closure element (7) is not retained in the retention position by the retention means (5).

4. Stopper device (1) according to one of the preceding claims, **characterized in that** the closure element (7) is - in the second functional state - arranged freely movable in the medicament container (45).

5. Stopper device (1) according to one of the preceding claims, **characterized in that** - at least in an ejection state of the stopper device (1) - the discharge projection (63), with a side and/or tip facing the closure element (7), is positioned against the closure element (7).

6. Stopper device (1) according to one of the preceding claims, **characterized in that** the closure element (7) has at least one first locking means, wherein the retention means (23) of the stopper device (1) has at least one second locking means embodied corresponding to the first locking means and arranged in the retention region (23) on a side of the stopper element (3) facing the closure element (7), wherein - in the first functional state - the first locking means and the second locking means engage in one another in a locking manner.

7. Stopper device (1) according to one of the preceding claims, **characterized in that** the stopper device (1) has an insertion sleeve (15) enveloping the stopper element (3), wherein an outer side of a sleeve wall is preferably embodied for tight contact against an inner wall (53) of the medicament container (45).

8. Stopper device (1) according to claim 7, **characterized in that** the insertion sleeve (15) is designed as a finger rest and has at least one radially protruding finger plate (19) at a proximal end, which finger plate (19) can be grasped from behind by a finger.

9. Stopper device (1) according to claim 7 or 8, **characterized in that** the insertion sleeve (15) has a fastening structure (41) for fastening the insertion sleeve (15) to the medicament container (45), wherein the fastening structure (41) preferably has a receptacle (43) for the medicament container (45), in which receptacle (43) the proximal end (49) of the medicament container (45) can be received in a locking and/or clamping manner.

10. Medicament container with a base body (47) and a stopper device (1) according to one of the preceding claims, wherein the stopper device (1) is arranged moved into the medicament container (45) such that the stopper element (3) seals off a proximal end (49) of the medicament container (45) with respect to a chamber (51) of the base body (47), wherein the chamber (51) is delimited in the radial direction by an inner wall (53) of the base body (47) and in the distal direction by an outlet opening (55) of the base body (47) and/or a closure cap (57).

11. Medicament container according to claim 10, **characterized in that** the outlet opening (55) of the base body (47) has a geometry, in particular a cruciform cross-section (60), at least on its side facing the chamber (51), which differs from a side of the closure element (7) facing the outlet opening such that a fluidically tight closure of the outlet opening (55) is prevented by the closure element (7).

12. Medicament container according to claim 10 or 11, **characterized by** a dispensing device (67), wherein a projection (73) is provided at a distal end (71) of the dispensing device (67).

13. Medicament container according to claim 12, **characterized in that** the stopper element (3) is elastic, wherein it is deformable by means of the projection (73) of the dispensing device (67), in particular using manual exertion of pressure on the dispensing device (67) in the distal direction, wherein the discharge projection (63) of the stopper element (3) has a greater strength in the axial direction than the pressure required to move the closure element (7).

14. Method for mixing two substances (31, 59) stored separately from one another in terms of flow in a medicament container (45) according to one of claims 10 to 13 with a stopper device (1) according to one of claims 1 to 9, wherein a first substance (31) of the two substances (31,59) is arranged in the cavity (29) of the stopper device (1) and a second substance (59) of the two substances (31,59) is arranged in the chamber (51) of the base body (47), wherein the stopper element (3) is acted upon with a force in the distal direction, wherein the fluidic separation of the two substances (31, 59) is eliminated, **characterized in that** the stopper device (1), in particular the stopper element (3), is deformed, at least partially, due to the distal force application, such that the closure element (7) is moved out of the retention position.

15. Method according to claim 14, **characterized in that** the stopper element (3) is displaced in the distal direction, wherein the fluidic separation of the two substances (31, 59) is eliminated, wherein the retention region (23) relaxes radially during the distal displacement of the stopper device (1), and wherein the retention of the closure element (7) in the retention position is released.

16. Method according to claim 14 or 15, **characterized in that** the medicament container (45) is shaken after the cavity (29) has been opened.

17. Method according to one of claims 14 to 16, **characterized in that** the stopper device (1) is displaced into an end position for the complete discharge of the two substances (31, 59) in the medicament container (45), wherein the closure element (7) is received in the end position in the cavity (29), and wherein the cavity (29) is completely filled by the closure element (7).

## Revendications

1. Dispositif bouchon (1) pour l'étanchéification d'un récipient pour médicaments (45), avec un élément bouchon (3), le dispositif bouchon (1) étant conçu pour étanchéifier le récipient pour médicaments (45) dans une direction axiale d'un axe médian (M) du récipient pour médicaments (45), le dispositif bouchon (1) étant conçu pour être au moins en partie insérable dans le récipient pour médicaments (45) et l'élément bouchon (3) étant conçu pour être déplaçable le long de l'axe médian (M) du récipient pour médicaments (45) lorsqu'il se trouve dans la position de montage prévue dans le récipient pour médicaments (45), le dispositif bouchon (1) comprenant une zone de retenue (23) avec au moins un moyen de retenue (5), dans lequel un élément de fermeture (7) - dans un premier état de fonctionnement du dispositif bouchon (1) - est retenu de manière amovible dans une position de retenue au moyen du moyen de retenue (5) de manière fixe par rapport à l'élément bouchon (3), en particulier sur l'élément bouchon (3), de telle sorte qu'est constituée une cavité (29) entre l'élément de fermeture (7) d'une part et l'élément bouchon (3) d'autre part, dans lequel l'élément de fermeture (7) est conçu sous la forme d'un corps mixte sphérique pour le récipient pour médicaments (45), **caractérisé en ce que** l'élément bouchon (3) - dans le premier état de fonctionnement - comprend, sur sa face tournée vers l'élément de fermeture (7), une saillie de distribution (63) orientée au moins en partie dans la direction de l'élément de fermeture (7).

2. Dispositif bouchon (1) selon la revendication 1, **caractérisé en ce que,** dans le premier état de fonctionnement, la cavité (29) est étanchéifiée fluidiquement contre une paroi, en particulier une paroi interne (53), du récipient pour médicaments (45).

3. Dispositif bouchon (1) selon l'une des revendications précédentes, **caractérisé en ce que,** dans un deuxième état de fonctionnement, la cavité (29) est ouverte, dans lequel l'élément de fermeture (7) n'est pas retenu dans la position de retenue par le moyen de retenue (5).

4. Dispositif bouchon (1) selon l'une des revendications précédentes, **caractérisé en ce que** l'élément de fermeture (7) - dans le deuxième état de fonctionnement - est disposé librement mobile dans le récipient pour médicaments (45).

5. Dispositif bouchon (1) selon l'une des revendications précédentes, **caractérisé en ce que** la saillie de distribution (63) - au moins dans un état d'éjection du dispositif bouchon (1) - repose contre l'élément de fermeture (7) avec une face et/ou un sommet tournés vers l'élément de fermeture (7).

6. Dispositif bouchon (1) selon l'une des revendications précédentes, **caractérisé en ce que** l'élément de fermeture (7) comprend au moins un premier moyen d'encliquetage, dans lequel le moyen de retenue (23) du dispositif bouchon (1) comprend au moins un deuxième moyen d'encliquetage conçu de manière correspondante au premier moyen d'encliquetage, lequel deuxième moyen d'encliquetage est disposé dans la zone de retenue (23) sur une face tournée vers l'élément de fermeture (7) de l'élément bouchon (3), dans lequel le premier moyen d'encliquetage et le deuxième moyen d'encliquetage - dans le premier état de fonctionnement - se mettent en prise l'un avec l'autre par encliquetage.

7. Dispositif bouchon (1) selon l'une des revendications précédentes, **caractérisé en ce que** le dispositif bouchon (1) comprend un manchon d'emboîtement (15) enserrant l'élément bouchon (3), dans lequel, de préférence, une face externe d'une paroi de manchon est conçue pour le contact étanche contre une paroi interne (53) du récipient pour médicaments (45).

8. Dispositif bouchon (1) selon la revendication 7, **caractérisé en ce que** le manchon d'emboîtement (15) est conçu sous la forme d'un repose-doigts et comprend au niveau d'une extrémité proximale au moins une plaque à doigts (19) faisant saillie radialement, laquelle est conçue pour être saisissable par l'arrière par un doigt.

9. Dispositif bouchon (1) selon l'une des revendications 7 ou 8, **caractérisé en ce que** le manchon d'emboîtement (15) comprend une structure de fixation (41) pour la fixation du manchon d'emboîtement (15) au récipient pour médicaments (45), dans lequel la structure de fixation (41) comprend de préférence un logement (43) pour le récipient pour médicaments (45), dans lequel logement le récipient pour médicaments (45) peut être reçu avec son extrémité proximale (49) par encliquetage et/ou par serrage.

10. Récipient pour médicaments avec un corps de base (47) et un dispositif bouchon (1) selon l'une des revendications précédentes, dans lequel le dispositif bouchon (1) est disposé de manière à être déplacé vers l'intérieur du récipient pour médicaments (45) par endroits de telle sorte que l'élément bouchon (3) étanchéifie une extrémité proximale (49) du récipient pour médicaments (45) par rapport à une chambre (51) du corps de base (47), dans lequel la chambre (51) est délimitée dans la direction radiale par une paroi interne (53) du corps de base (47) et dans la direction distale par une ouverture de sortie (55) du corps de base (47) et/ou un capuchon de fermeture (57).

11. Récipient pour médicaments selon la revendication 10, **caractérisé en ce que** l'ouverture de sortie (55) du corps de base (47), au moins sur sa face tournée vers la chambre (51), présente une géométrie, en particulier une section transversale cruciforme (60), qui diffère d'une face de l'élément de fermeture (7) tournée vers l'ouverture de sortie de telle sorte qu'une fermeture fluidiquement étanche de l'ouverture de sortie (55) est empêchée par l'élément de fermeture (7).

12. Récipient pour médicaments selon la revendication 10 ou la revendication 11, **caractérisé par** un dispositif de distribution (67), dans lequel une protubérance (73) est prévue au niveau d'une extrémité distale (71) du dispositif de distribution (67).

13. Récipient pour médicaments selon la revendication 12, **caractérisé en ce que** l'élément bouchon (3) est élastique, dans lequel il est déformable au moyen de la protubérance (73) du dispositif de distribution (67), en particulier par exercice d'une pression manuelle dans la direction distale sur le dispositif de distribution (67), dans lequel la saillie de distribution (63) de l'élément bouchon (3) présente une résistance dans la direction axiale qui est supérieure à celle qui correspond à la pression nécessaire pour la distribution de l'élément de fermeture (7).

14. Procédé pour le mélange de deux substances (31, 59) stockées séparément l'une de l'autre d'un point de vue fluidique dans un récipient pour médicaments (45) selon l'une des revendications 10 à 13 avec un dispositif bouchon (1) selon l'une des revendications 1 à 9, dans lequel une première substance (31) des deux substances (31, 59) est disposée dans la cavité (29) du dispositif bouchon (1) et une deuxième substance (59) des deux substances (31, 59) est disposée dans la chambre (51) du corps de base (47), dans lequel l'élément bouchon (3) est soumis à une force dans la direction distale, dans lequel la séparation fluidique des deux substances (31, 59) est supprimée, **caractérisé en ce que** le dispositif bouchon (1), en particulier l'élément bouchon (3), est déformé, au moins en partie, en raison de l'application de la force distale, dans une mesure telle que l'élément de fermeture (7) est déplacé hors de la position de retenue.

15. Procédé selon la revendication 14, **caractérisé en ce que** l'élément bouchon (3) est déplacé dans la direction distale, dans lequel la séparation fluidique des deux substances (31, 59) est supprimée, dans lequel la zone de retenue (23) se détend radialement pendant le déplacement distal du dispositif bouchon (1), et dans lequel la retenue de l'élément de fermeture (7) dans la position de retenue est relâchée.

16. Procédé selon la revendication 14 ou la revendication 15, **caractérisé en ce que** le récipient pour médicaments (45) est agité après l'ouverture de la cavité (29).

17. Procédé selon l'une des revendications 14 à 16, **caractérisé en ce que** le dispositif bouchon (1) pour la distribution complète des deux substances (31, 59) dans le récipient pour médicaments (45) est déplacé dans une position finale, dans lequel l'élément de fermeture (7) est reçu dans la cavité (29) dans la position finale, et dans lequel la cavité (29) est complètement remplie par l'élément de fermeture (7).
